# EUROPEAN PATENT SPECIFICATION

(11) **EP 4 329 862 B1**
(45) Date of publication and mention of the grant of the patent: **21.10.2026**
(21) Application number: 22736050.0
(22) Date of filing: 20.05.2022
(51) Int. Cl.: A61B 17/3209, A61M 25/06, A61M 25/00, A61M 25/09

(54) **RAPIDLY INSERTABLE CENTRAL CATHETERS, INTRODUCERS, AND INSERTION DEVICES INCLUDING COMBINATIONS**
SCHNELL EINSETZBARE ZENTRALKATHETER, EINFÜHRVORRICHTUNGEN UND EINFÜHRVORRICHTUNGEN MIT KOMBINATIONEN
CATHÉTERS CENTRAUX À INSERTION RAPIDE, INTRODUCTEURS ET DISPOSITIFS D'INSERTION COMPRENANT DES COMBINAISONS

(30) Priority: 20.05.2021 US 202163191207 P; 30.11.2021 US 202163284533 P
(43) Date of publication of application: 06.03.2024
(73) Proprietor: Bard Access Systems, Inc., Salt Lake City, UT 84116 (US)
(72) Inventor: BLANCHARD, Daniel, B., Bountiful, UT 84010 (US); HOWELL, Glade, H., Draper, UT 84020 (US); SPATARO, Joe, Cottonwood Heights, UT 84121 (US); THORNLEY, Kyle, G., Farmington, UT 84025 (US); MCKINNON, Austin, J., Heriman, UT 84096 (US)
(74) Representative: Hoffmann Eitle
(86) International application number: PCT/US2022/030365
(87) International publication number: WO 2022/246271

(56) References cited:
- US-A1- 2018 043 138
- US-A1- 2019 038 113
- US-A1- 2021 121 661

## Description

### PRIORITY

This application claims the benefit of priority to U.S. Provisional Patent Application No. 63/191,207, filed May 20, 2021, and U.S. Provisional Patent Application No. 63/284,533, filed November 30, 2021.

### BACKGROUND

Central venous catheter ("CVCs") are commonly introduced into patients and advanced through their vasculatures by way of the Seldinger technique. The Seldinger technique utilizes a number of steps and medical devices (e.g., a needle, a scalpel, a guidewire, an introducer sheath, a dilator, a CVC, etc.). While the Seldinger technique is effective, the number of steps are time consuming, handling the number of medical devices is awkward, and both of the foregoing can lead to patient trauma. In addition, there is a relatively high potential for touch contamination due to the number of medical devices that need to be interchanged during the number of steps of the Seldinger technique. As such, there is a need to reduce the number of steps and medical devices involved in introducing a catheter such as a CVC into a patient and advancing the catheter through a vasculature thereof.
US 2021/121661 A1 relates to a rapidly insertable central catheter including a catheter tube, a suture wing disposed over a medial portion of the catheter tube, a hub coupled to a proximal-end portion of the catheter tube, and a number of extension legs extending from the hub equal to a number of lumens extending through the rapidly insertable central catheter.

Disclosed herein are rapidly insertable central catheters ("RICCs"), introducers, and insertion devices including combinations thereof that address the foregoing.

### SUMMARY

The invention is according to claim 1. Preferred embodiments of the invention are set forth in the dependent claims. Disclosed herein is a RICC insertion system including a RICC assembly, a RICC insertion device, and an introducer. The RICC assembly includes a RICC, an access guidewire, and a keeper. The RICC includes a catheter tube, a catheter hub coupled to a proximal portion of the catheter tube, and one or more extension legs. Each extension leg of the one-or-more extension legs is coupled to the catheter hub by a distal portion thereof. The access guidewire is disposed in a primary lumen of the RICC. The keeper includes a splittable casing over both the catheter tube and a distal portion of the access guidewire extending from a distal end of the RICC forming a longitudinal composite thereof. The RICC insertion device includes a frame, one or more roller wheels, and a nose cover over a nose of the frame. The frame includes a longitudinal handle, the nose, and a curved cradle. The handle includes a wheel well. The nose extends from a distal portion of the handle distal of the wheel well. The nose includes a through channel configured for advancing the catheter tube of the RICC therethrough. The cradle extends from a proximal portion of the handle. The one-or-more roller wheels are disposed in the wheel well. The nose cover and nose form a split channel configured for both splitting and passing the splittable casing therethrough. The RICC insertion device is configured for advancing the RICC assembly from an initial position in the frame to a final position in the frame. This is accomplished by repeatedly pushing the longitudinal composite into the one-or-more roller wheels and rolling the longitudinal composite across the one-or-more roller wheels.

In some embodiments, the keeper further includes a catheter-hub holder to which a proximal end of the splittable casing is attached. The catheter-hub holder is configured to hold the catheter hub therein as well as keep the splittable casing in position over the catheter tube and the access guidewire.

In some embodiments, the catheter-hub holder includes a perimetrical wall around at least a portion of a perimeter of the catheter-hub holder. The perimetrical wall defines a recess into which the catheter hub fits with an engineering fit.

In some embodiments, the cradle includes a curved extension over a distal portion of the cradle. The extension substantially follows a same curve as the cradle.

In some embodiments, the cradle includes an enclosure over the distal portion of the cradle with the extension extending therefrom. The enclosure is configured to enclose therein an otherwise exposed proximal portion of the access guidewire in the initial position of the RICC assembly in the frame.

In some embodiments, the RICC insertion device further includes a retaining clip. The retaining clip is configured to clip on to a proximal portion of the RICC and retain the RICC assembly in the RICC insertion device. The retaining clip is also configured to keep the catheter tube from prematurely advancing over the access guidewire. The retaining clip includes a post configured to engage with a slot in the extension that faces an open face of the cradle.

In some embodiments, the retaining clip is configured to disengage with the slot in the extension when any remaining slack of the longitudinal composite is removed upon advancing the RICC assembly from the initial position to the final position in the frame.

In some embodiments, the frame further includes a retaining arch over the cradle proximate the proximal portion of the handle from which the cradle extends. The retaining arch is configured to retain the RICC assembly in the RICC insertion device. The retaining arch is also configured to hold the longitudinal composite over the handle as the RICC assembly transitions over the proximal portion of the handle from which the cradle extends upon advancing the RICC assembly from the initial position to the final position in the frame.

In some embodiments, the nose cover and the nose further form a retaining clamp configured to slidably clamp the longitudinal composite therein. The nose cover is rotatable over the nose. The nose cover includes a longitudinal gap configured to rotatably align with the through channel in the nose for removal of the longitudinal composite, the splittable casing, or the catheter tube therefrom.

In some embodiments, the RICC insertion device further includes a retractable nicking blade. The retractable nicking blade is disposed in a nicking-blade carriage slidably integrated into the nose. The nicking-blade carriage has a nicking position with the retractable nicking blade extending beyond a distal end of the nose. The nicking-blade carriage also has a safety position with the retractable nicking blade short of the distal end of the nose.

In some embodiments, the introducer includes an introducer needle and a splittable introducer sheath. The introducer needle includes a needle shaft and a needle hub. The needle shaft includes a needle tip in a distal portion of the needle shaft. The needle hub is coupled to a proximal portion of the needle shaft. The introducer sheath is configured to accept therein the introducer needle. The introducer sheath includes a splittable sheath body and a splittable sheath hub coupled to a proximal portion of the sheath body. The sheath hub includes a pair of outwardly extending wings along a length of the sheath hub. The wings have an internal angle of about 90° or less between the wings. The wings are configured for splitting the sheath hub by pinching the wings together with a single hand.

In some embodiments, the sheath hub includes a single longitudinal fault along a side of the sheath hub opposite a vertex of the internal angle formed by the wings. The sheath hub is configured to split along the fault for propagation along a same side of the sheath body when the wings are pinched together.

In some embodiments, the sheath hub includes a pair of longitudinal faults including a primary fault and a secondary fault. The primary fault extends along a primary side of the sheath hub opposite a vertex of the internal angle formed by the wings. The secondary fault extends along a secondary side of the sheath hub opposite the primary fault. The sheath hub is configured to split along the primary fault for propagation along the primary side of the sheath body when the wings are pinched together. The sheath hub is also configured to split along the secondary fault for propagation along the secondary side of the sheath body when the wings are pulled apart.

In some embodiments, the sheath hub further includes a valved cap disposed in a proximal portion of the sheath hub. The valved cap includes a tapered female valved-cap connector and a septum distal of a proximal opening in the female valved-cap connector. The female valved-cap connector is configured to accept therein a tapered male needle-hub connector extending from a distal portion of the needle hub. The septum is configured to accept therethrough the needle shaft.

In some embodiments, the valved cap is partially or fully split such that the valved cap splits with the sheath hub when the wings are pinched together.

In some embodiments, the sheath hub further includes an overmolded nicking blade and a hinged nicking-blade cover. The overmolded nicking blade distally extends from the sheath hub. The nicking-blade cover has an opened state with the nicking-blade cover opened away from the overmolded nicking blade. The nicking-blade cover also has a closed state with the nicking-blade cover closed over the overmolded nicking blade.

In some embodiments, the introducer further includes a syringe. The syringe includes a tapered male syringe tip extending from a distal portion of the syringe. The syringe tip is configured to insert into a tapered female needle-hub connector in a proximal portion of the needle hub.

Also disclosed herein is a RICC insertion assembly including, in some embodiments, a RICC assembly and a RICC insertion device. The RICC assembly includes a RICC, an access guidewire, and a keeper. The RICC includes a catheter tube, a catheter hub coupled to a proximal portion of the catheter tube, and one or more extension legs. Each extension leg of the one-or-more extension legs is coupled to the catheter hub by a distal portion thereof. The access guidewire is disposed in a primary lumen of the RICC. The keeper includes a splittable casing over both the catheter tube and a distal portion of the access guidewire extending from a distal end of the RICC forming a longitudinal composite thereof. The RICC insertion device includes a frame, one or more roller wheels, and a nose cover over a nose of the frame. The frame includes a longitudinal handle, the nose, and a curved cradle. The handle includes a wheel well. The nose extends from a distal portion of the handle distal of the wheel well. The nose includes a through channel configured for advancing the catheter tube of the RICC therethrough. The cradle extends from a proximal portion of the handle. The one-or-more roller wheels are disposed in the wheel well. The nose cover and nose form a split channel configured for both splitting and passing the splittable casing therethrough. The RICC insertion device is configured for advancing the RICC assembly from an initial position in the frame to a final position in the frame. This is accomplished by repeatedly pushing the longitudinal composite into the one-or-more roller wheels and rolling the longitudinal composite across the one-or-more roller wheels.

In some embodiments, a distal end of the longitudinal composite, the splittable casing, or the access guidewire is substantially commensurate with a distal end of the frame in the initial position of the RICC assembly in the frame.

In some embodiments, a slack loop of the longitudinal composite extends away from both the handle and the cradle in the initial position of the RICC assembly in the frame.

In some embodiments, the longitudinal composite is substantially split in the final position of the RICC assembly in the frame.

In some embodiments, a proximal end of an extension leg or a Luer connector coupled to the extension leg of the one-or-more extension legs of the RICC is substantially commensurate with a proximal end of the cradle in the initial position of the RICC assembly in the frame.

In some embodiments, a proximal end of the access guidewire is coupled to the proximal end of the cradle in the initial position of the RICC assembly in the frame.

In some embodiments, the one-or-more extension legs of the RICC lie substantially over the handle in the final position of the RICC assembly in the frame.

In some embodiments, the catheter hub of the RICC lies over the wheel well in the final position of the RICC assembly in the frame.

Also disclosed herein is a method, which is not part of the invention, for inserting a RICC into a blood-vessel lumen of a patient. The method includes, in some embodiments, an obtaining step, a needle tract-establishing step, an introducer needle-removing step, an access guidewire-advancing step, an introducer sheath-removing step, and a catheter tube-advancing step. The obtaining step includes obtaining a RICC insertion system. The RICC insertion system includes an introducer, a RICC insertion device, and a RICC assembly including the RICC. The RICC assembly is optionally already disposed in the RICC insertion device in a substantially ready-to-operate state of a RICC insertion assembly. The needle tract-establishing step includes establishing a needle tract from an area of skin into the blood-vessel lumen of the patient with an introducer needle of the introducer. The introducer needle is disposed in a splittable introducer sheath of the introducer for the establishing of the needle tract. The introducer needle-removing step includes removing the introducer needle from the introducer sheath leaving the introducer sheath in the blood-vessel lumen. The access guidewire-advancing step includes advancing an access guidewire through a nose and nose cover of the RICC insertion device, through a splittable sheath hub of the introducer, through the introducer sheath, and into the blood-vessel lumen. The access guidewire-advancing step is accomplished, in part, by splitting a splittable casing away from a longitudinal composite of the RICC assembly. A distal portion of the access guidewire that extends from a primary-lumen aperture of a catheter tube of the RICC is freed from the longitudinal composite with the splitting of the splittable casing for the advancing of the access guidewire. The introducer sheath-removing step includes removing the introducer sheath leaving the access guidewire in the blood-vessel lumen. The catheter tube-advancing step includes advancing the catheter tube over the access guidewire and into the blood-vessel lumen. The catheter tube-advancing step is accomplished, in part, by further splitting the splittable casing away from the longitudinal composite of the RICC assembly. The catheter tube is freed from the longitudinal composite with the splitting of the splittable casing, which allows the catheter tube to be advanced over the access guidewire upon removal of any remaining slack of the longitudinal composite in the RICC insertion assembly.

In some embodiments, needle tract-establishing step includes ensuring blood flashes back into a needle hub of the introducer needle for confirmation the needle tract extends into the blood-vessel lumen.

In some embodiments, the method, which is not part of the invention, further includes a blood-aspirating step. The blood-aspirating step includes aspirating blood with a syringe coupled to the introducer needle for confirmation the needle tract extends into the blood-vessel lumen. The blood-aspirating step is performed before the introducer needle-removing step.

In some embodiments, the method, which is not part of the invention, further includes an introducer sheath-advancing step. The introducer sheath-advancing step includes advancing the introducer sheath over the introducer needle and farther into the blood-vessel lumen. The introducer sheath-advancing step is performed before the introducer needle-removing step.

In some embodiments, the method, which is not part of the invention, further includes a connector-connecting step The connector-connecting step includes inserting a male nose connector of the nose of the RICC insertion device into a female valved-cap connector of the sheath hub of the introducer sheath. The connector-connecting step is performed before the access guidewire-advancing step.

In some embodiments, the method, which is not part of the invention, further includes a skin-nicking step. The skin-nicking step includes nicking the area of skin around the needle tract with a nicking blade extending from the sheath hub. The skin-nicking step is performed when the catheter tube is 2.33 mm (7 Fr) or larger. The skin-nicking step is also performed before the access guidewire-advancing step.

In some embodiments, the method, which is not part of the invention, further includes a cover-opening step and a cover-closing step. The cover-opening step includes opening a hinged nicking-blade cover away from the nicking blade and into a nicking position away from the nicking blade. The cover-opening step is performed before the skin-nicking step. The cover-closing step includes closing the nicking-blade cover over the nicking blade into a safety position over the nicking blade. The cover-closing step is performed after the skin-nicking step.

In some embodiments, the method, which is not part of the invention, further includes an alternative skin-nicking step. The alternative skin-nicking step includes nicking the area of skin around the needle tract with a retractable nicking blade integrated into the nose of the RICC insertion device. The alternative skin-nicking step is performed when the catheter tube is 7 Fr or larger. The alternative skin-nicking step is also performed before the catheter tube-advancing step.

In some embodiments, the method, which is not part of the invention, further includes a pair of carriage-sliding steps including a first carriage-sliding step and a second carriage-sliding step. The first carriage-sliding step includes distally sliding a nicking-blade carriage including the nicking blade overmolded therein into a nicking position such that the nicking blade extends beyond a distal end of the nose of the RICC insertion device. The second carriage-sliding step includes proximally sliding the nicking-blade carriage into a safety position such that the nicking blade is short of the distal end of the nose of the RICC insertion device.

In some embodiments, the introducer sheath-removing step includes splitting the introducer sheath to form a split along a side of the introducer sheath by pinching together a pair of wings outwardly extending from the sheath hub. The introducer sheath-removing step also includes propagating the split in a side of the sheath hub along a same side of a sheath body of the introducer.

In some embodiments, the access guidewire-advancing step or the catheter tube-advancing step includes repeatedly pushing the longitudinal composite into one or more roller wheels disposed in a wheel well of a longitudinal handle of the RICC insertion device and rolling the longitudinal composite across the one-or-more roller wheels with a single hand to split the splittable casing away from the longitudinal composite.

In some embodiments, the access guidewire-advancing step or the catheter tube-advancing step includes pulling the splittable casing of the longitudinal composite out of a split channel formed between the nose of the RICC insertion device and the nose cover thereover to split the splittable casing away from the longitudinal composite.

In some embodiments, a retaining clip clipped on to a proximal portion of the RICC disengages with a curved extension over a cradle of the RICC insertion device upon removal of any remaining slack of the longitudinal composite in the RICC insertion assembly, thereby allowing the catheter tube to advance over the access guidewire.

In some embodiments, the method, which is not part of the invention, further includes an access guidewire-removing step. The access guidewire-removing step includes removing the access guidewire leaving the catheter tube in the blood-vessel lumen.

In some embodiments, the method, which is not part of the invention, further includes a keeper-removing step. The keeper-removing step includes removing a keeper including a proximal end of the splittable casing attached to a catheter-hub holder from the RICC assembly. The keeper-removing step is accomplished, in part, by removing a catheter hub of the RICC from the catheter-hub holder and splitting any remaining splittable casing of the longitudinal composite away from the catheter tube.

In some embodiments, the method, which is not part of the invention, further includes a catheter tube-freeing step.
The catheter tube-freeing step includes freeing the catheter tube of the RICC from a retaining clamp formed of the nose and nose cover of the RICC insertion device. The catheter tube-freeing step is accomplished, in part, by rotating the nose cover over the nose to align a longitudinal gap of the nose cover with a through channel in the nose and pulling the catheter tube away from the RICC insertion device.

Also disclosed herein is a catheter insertion system for a RICC assembly including, in some embodiments, an insertion assembly having a housing including a blood-flash nozzle and a catheter nozzle; a blood-flash indicator in fluid communication with the blood-flash nozzle; a RICC disposed within an interior cavity of the housing and slidably engaged with the catheter nozzle; and an introducer including an introducer needle and an introducer sheath, the introducer releasably engaged with one of the blood-flash nozzle or a catheter nozzle.

In some embodiments, the catheter insertion system further includes a needle-retraction system coupled with the introducer needle. The needle-retraction system is configured to withdraw the introducer needle from the introducer sheath proximally through the blood-flash nozzle.

In some embodiments, the blood-flash indicator is in fluid communication with a needle lumen of the needle.

In some embodiments, a sheath hub of the introducer sheath is configured to releasably engage the catheter nozzle and longitudinally align a sheath lumen of the sheath with the RICC.

In some embodiments, the catheter insertion system further includes a catheter-advancement assembly configured to slidably engage with the RICC with the catheter nozzle.

In some embodiments, the catheter insertion system further includes an access guidewire coupled to a guidewire-advancement assembly. The guidewire-advancement assembly is configured to slidably engage the access guidewire with the catheter nozzle.

Also disclosed herein is a method of placing a catheter, which is not part of the invention, within a vasculature of
a patient including, in some embodiments, accessing a vasculature with an introducer needle of an introducer; withdrawing the introducer needle from a sheath lumen of an introducer sheath of the introducer through a blood-flash nozzle of an insertion assembly; detaching a sheath hub of the introducer sheath from the blood-flash nozzle and engaging the sheath hub with a catheter nozzle of the insertion assembly; advancing an access guidewire through the catheter nozzle and the sheath lumen; splitting the introducer sheath longitudinally to disengage the introducer sheath from the access guidewire; and advancing the catheter through the catheter nozzle over the access guidewire.

In some embodiments, the method, which is not part of the invention, further includes actuating a blood-flash
indicator to draw a fluid flow proximally through a needle lumen of the introducer needle and through the blood-flash nozzle.

In some embodiments, the method, which is not part of the invention, further includes actuating a needle-retraction
assembly, disposed within the insertion assembly, to withdraw the introducer needle through the blood-flash nozzle and into an interior cavity of the insertion assembly.

In some embodiments, the catheter includes one of a rapidly insertable central catheter, a central venous catheter, a peripherally inserted central catheter, or a dialysis catheter.

These and other features of the concepts provided herein will become more apparent to those of skill in the art in view of the accompanying drawings and following description, which describe particular embodiments of such concepts in greater detail.

### DRAWINGS

FIG. 1 provides a RICC insertion system including a RICC insertion assembly and an introducer in accordance with some embodiments.
FIG. 2 provides a RICC in accordance with some embodiments.
FIG. 3 provides a distal portion of the RICC in accordance with some embodiments.
FIG. 4 provides a first transverse cross section of the RICC in accordance with some embodiments.
FIG. 5 provides a second or third transverse cross section of the RICC in accordance with some embodiments.
FIG. 6 provides a RICC assembly in accordance with some embodiments.
FIG. 7 provides a first transverse cross section of the RICC assembly in accordance with some embodiments.
FIG. 8 provides a second transverse cross section of the RICC assembly in accordance with some embodiments.
FIG. 9 provides a first alternative RICC assembly in accordance with some embodiments.
FIG. 10 provides a transverse cross section of the first alternative RICC assembly in accordance with some embodiments.
FIG. 11 provides a second alternative RICC assembly in accordance with some embodiments.
FIG. 12 provides a first transverse cross section of the second alternative RICC assembly in accordance with some embodiments.
FIG. 13 provides a second transverse cross section of the second alternative RICC assembly in accordance with some embodiments.
FIG. 14 provides a third alternative RICC assembly in accordance with some embodiments.
FIG. 15A provides a top view of a fourth alternative RICC assembly in accordance with some embodiments.
FIG. 15B provides a side view of the fourth alternative RICC assembly in accordance with some embodiments.
FIG. 16 provides a RICC insertion assembly including the RICC assembly disposed in a RICC insertion device in a ready-to-operate state of the RICC insertion assembly in accordance with some embodiments.
FIG. 17 provides a nose cover over a nose of the RICC insertion device in accordance with some embodiments.
FIG. 18A provides a retaining clamp formed between the nose cover and the nose of the RICC insertion device when the retaining clamp is in a closed state in accordance with some embodiments.
FIG. 18B provides the retaining clamp when the retaining clamp is in an open state in accordance with some embodiments.
FIG. 19 provides a curved extension of a curved cradle of the RICC insertion device including a slot configured to engage with the retaining clip in accordance with some embodiments.
FIG. 20 provides a retaining clip of the RICC insertion device in accordance with some embodiments.
FIG. 21A provides a retractable nicking blade integrated into the nose of the RICC insertion device when the nicking blade is in a safety position in accordance with some embodiments.
FIG. 21B provides the retractable nicking blade when the nicking blade is in a nicking position in accordance with some embodiments.
FIG. 22 provides the RICC insertion assembly with the RICC assembly in an earlier intermediate position in a frame of the RICC insertion device in accordance with some embodiments.
FIG. 23A provides the RICC insertion assembly with the RICC assembly in a same or different earlier intermediate position in the frame of the RICC insertion device in which a slack loop of a longitudinal composite remains in the RICC insertion assembly in accordance with some embodiments.
FIG. 23B provides the RICC insertion assembly with the RICC assembly in a later intermediate position in the frame of the RICC insertion device in which no slack loop of the longitudinal composite remains in the RICC insertion assembly in accordance with some embodiments.
FIG. 24 provides the RICC insertion assembly with the RICC assembly in the final position in the frame of the RICC insertion device assembly in accordance with some embodiments.
FIG. 25 provides an introducer in accordance with some embodiments.
FIG. 26 provides a pair of outwardly extending wings along a length of a sheath hub of the introducer in accordance with some embodiments.
FIG. 27 provides a longitudinal cross section of the sheath hub in accordance with some embodiments.
FIG. 28A provides an overmolded nicking blade integrated into the sheath hub with a nicking-blade cover in a safety position in accordance with some embodiments.
FIG. 28B provides the overmolded nicking blade integrated into the sheath hub with the nicking-blade cover in a nicking position in accordance with some embodiments.
FIG. 29 provides another RICC insertion system including another RICC insertion assembly in accordance with some embodiments.
FIG. 30 provides a detailed view of an introducer of the RICC insertion assembly of FIG. 29 in accordance with some embodiments.
FIG. 31 provides a detailed view of a nose portion of a RICC insertion device of the RICC insertion assembly of FIG. 29 in accordance with some embodiments.
FIG. 32A provides a first state of the RICC insertion assembly of FIG. 29 in a method of using the RICC insertion system, which is not part of the invention, in accordance with some embodiments.
FIG. 32B provides a second state of the RICC insertion assembly of FIG. 29 in the method of using the RICC insertion system, which is not part of the invention, in accordance with some embodiments.
FIG. 32C provides a third state of the RICC insertion assembly of FIG. 29 in the method of using the RICC insertion system, which is not part of the invention, in accordance with some embodiments.
FIG. 32D provides a fourth state of the RICC insertion assembly of FIG. 29 in the method of using the RICC insertion system, which is not part of the invention, in accordance with some embodiments.
FIG. 32E provides a fifth state of the RICC insertion assembly of FIG. 29 in the method of using the RICC insertion system, which is not part of the invention, in accordance with some embodiments.
FIG. 32F provides a sixth state of the RICC insertion assembly of FIG. 29 in the method of using the RICC insertion system, which is not part of the invention, in accordance with some embodiments.
FIG. 32G provides a seventh state of the RICC insertion assembly of FIG. 29 in the method of using the RICC insertion system, which is not part of the invention, in accordance with some embodiments.

### DESCRIPTION

Before some particular embodiments are disclosed in greater detail, it should be understood that the particular embodiments disclosed herein do not limit the scope of the concepts provided herein. It should also be understood that a particular embodiment disclosed herein can have features that can be readily separated from the particular embodiment and optionally combined with or substituted for features of any of a number of other embodiments disclosed herein.

Regarding terms used herein, it should also be understood the terms are for the purpose of describing some particular embodiments, and the terms do not limit the scope of the concepts provided herein. Ordinal numbers (e.g., first, second, third, etc.) are generally used to distinguish or identify different features or steps in a group of features or steps, and do not supply a serial or numerical limitation. For example, "first," "second," and "third" features or steps need not necessarily appear in that order, and the particular embodiments including such features or steps need not necessarily be limited to the three features or steps. In addition, any of the foregoing features or steps can, in turn, further include one or more features or steps unless indicated otherwise. Labels such as "left," "right," "top," "bottom," "front," "back," and the like are used for convenience and are not intended to imply, for example, any particular fixed location, orientation, or direction. Instead, such labels are used to reflect, for example, relative location, orientation, or directions. Singular forms of "a," "an," and "the" include plural references unless the context clearly dictates otherwise.

With respect to "proximal," a "proximal portion" or a "proximal-end portion" of, for example, a catheter includes a portion of the catheter intended to be near a clinician when the catheter is used on a patient. Likewise, a "proximal length" of, for example, the catheter includes a length of the catheter intended to be near the clinician when the catheter is used on the patient. A "proximal end" of, for example, the catheter includes an end of the catheter intended to be near the clinician when the catheter is used on the patient. The proximal portion, the proximal-end portion, or the proximal length of the catheter can include the proximal end of the catheter; however, the proximal portion, the proximal-end portion, or the proximal length of the catheter need not include the proximal end of the catheter. That is, unless context suggests otherwise, the proximal portion, the proximal-end portion, or the proximal length of the catheter is not a terminal portion or terminal length of the catheter.

With respect to "distal," a "distal portion" or a "distal-end portion" of, for example, a catheter includes a portion of the catheter intended to be near or in a patient when the catheter is used on the patient. Likewise, a "distal length" of, for example, the catheter includes a length of the catheter intended to be near or in the patient when the catheter is used on the patient. A "distal end" of, for example, the catheter includes an end of the catheter intended to be near or in the patient when the catheter is used on the patient. The distal portion, the distal-end portion, or the distal length of the catheter can include the distal end of the catheter; however, the distal portion, the distal-end portion, or the distal length of the catheter need not include the distal end of the catheter. That is, unless context suggests otherwise, the distal portion, the distal-end portion, or the distal length of the catheter is not a terminal portion or terminal length of the catheter.

Unless defined otherwise, all technical and scientific terms used herein have the same meaning as commonly understood by those of ordinary skill in the art.

As set forth above, there is a need to reduce the number of steps and medical devices involved in introducing a catheter such as a CVC into a patient and advancing the catheter through a vasculature thereof. Disclosed herein are RICCs, introducers, and insertion devices including combinations thereof that address the foregoing. However, it should be understood the RICCs are but one type of catheter in which the concepts provided herein can be embodied or otherwise incorporated. Indeed, peripherally inserted central catheters ("PICCs"), dialysis catheters, or the like can also embody or otherwise incorporate the concepts provided herein for the RICCs, introducers, and insertion devices including combinations thereof.

### RICC insertion systems

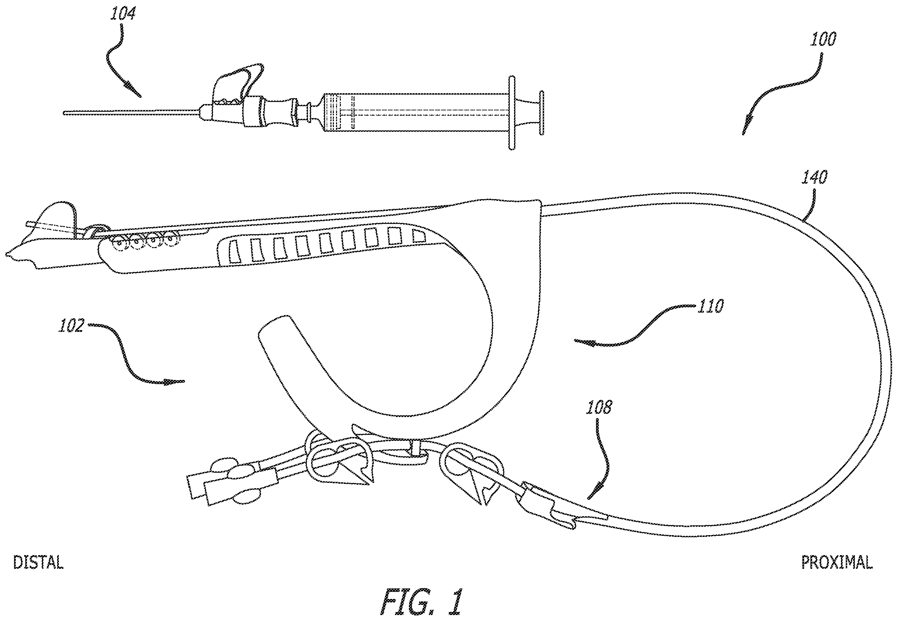

FIG. 1 provides a RICC insertion system 100 including a RICC insertion assembly 102 and an introducer 104 in accordance with some embodiments.

As shown, the RICC insertion system 100 can include a RICC 106 as part of a RICC assembly 108, a RICC insertion device 110, and the introducer 104. The RICC assembly 108 can be disposed in the initial position in the frame 172 of the RICC insertion device 110 as set forth below, thereby forming a substantially ready-to-operate state of the RICC insertion assembly 102 as further shown in FIG. 1.

For convenience, description for certain components of the RICC insertion system 100 including the RICC assembly 108, the RICC insertion device 110, the RICC insertion assembly 102, and the introducer 104 is set forth below in sections named for the certain components. However, description for a component of the RICC insertion system 100 is not limited to any particular section. Indeed, description for any component of the RICC insertion system 100 can crossover from one section into one or more other sections in view of the interrelatedness of the components of the RICC insertion system 100. For example, description for the RICC assembly 108 or the RICC insertion device 110 can crossover from the section therefor into the section for the RICC insertion assembly 102. Likewise, description for the RICC insertion assembly 102 can crossover from the section therefor into the section for the RICC assembly 108 or the RICC insertion device 110. Therefore, it should be understood that description for the RICC assembly 108, the RICC insertion device 110, the RICC insertion assembly 102, the introducer 104, or any other components of the RICC insertion system 100 might be found anywhere in this patent application.

### RICC insertion kits

While not shown, a RICC insertion kit can include the RICC insertion system 100, instructions for use of the RICC insertion system 100, and packaging. Again, the RICC insertion system 100 can include the RICC assembly 108, the RICC insertion device 110, and the introducer 104. The RICC assembly 108 can be disposed in the RICC insertion device 110 in the substantially ready-to-operate state of the RICC insertion assembly 102 for immediate use of the RICC insertion assembly 102 upon its removal from the RICC insertion kit. The RICC insertion system 100 can be packaged in the packaging (e.g., a polyethylene pouch, a polyethylene/Tyvek^{®} pouch, etc.). The instructions for use can be either packaged in the packaging with the RICC insertion system 100 or printed on the packaging.

Notwithstanding the foregoing RICC insertion kit, a combination of components less than an entirety of the RICC insertion system 100 (i.e., the RICC assembly 108, the RICC insertion device 110, and the introducer 104) can be packaged together in some packaging with instructions for use of the combination of components or the entirety of the RICC insertion system 100 in the packaging or printed on the packaging. For example, the RICC assembly 108 and the RICC insertion device 110 can be packaged in the substantially ready-to-operate state of the RICC insertion assembly 102 with instructions for use of the RICC insertion assembly 102 or the entirety of the RICC insertion system 100 in the packaging or printed on the packaging. Each component of the RICC insertion system 100 (i.e., the RICC assembly 108, the RICC insertion device 110, and the introducer 104) can also be separately packaged in some packaging with instructions for use of the component, a combination of components including the component, or the entirety of the RICC insertion system 100 in the packaging or printed on the packaging. For example, the introducer 104 can be packaged with instructions for use of the introducer 104 or the entirety of the RICC insertion system 100 in the packaging or printed on the packaging.

### RICC assemblies

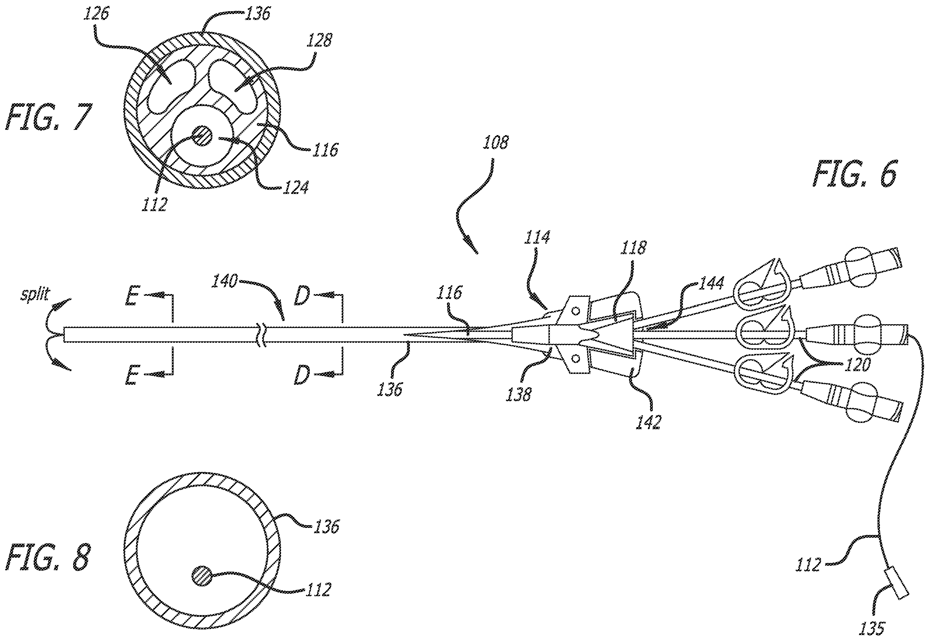

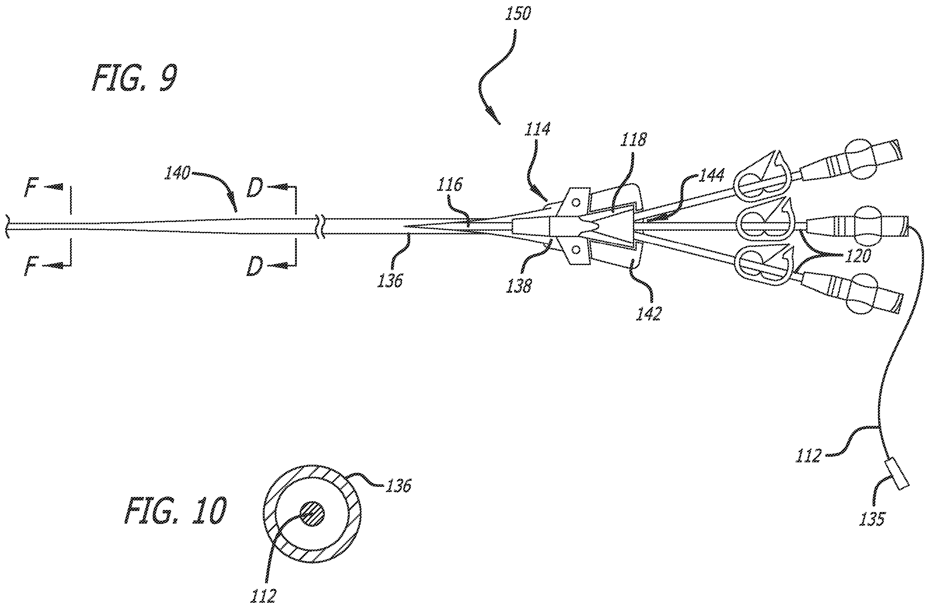

FIG. 6 provides the RICC assembly 108 in accordance with some embodiments, whereas FIGS. 9, 11, 14, 15A, and 15B provide some alternative RICC assemblies in accordance with some embodiments.

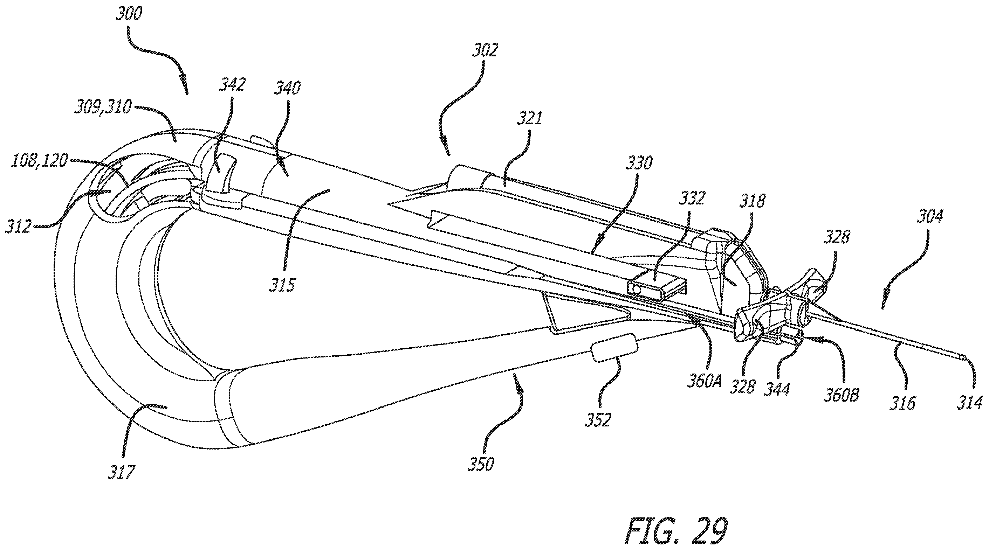

As shown, the RICC assembly 108 can include the RICC 106, an access guidewire 112, and a keeper 114. Notably, the RICC assembly 108 need not include the keeper 114 in certain embodiments such as that of FIG. 29.

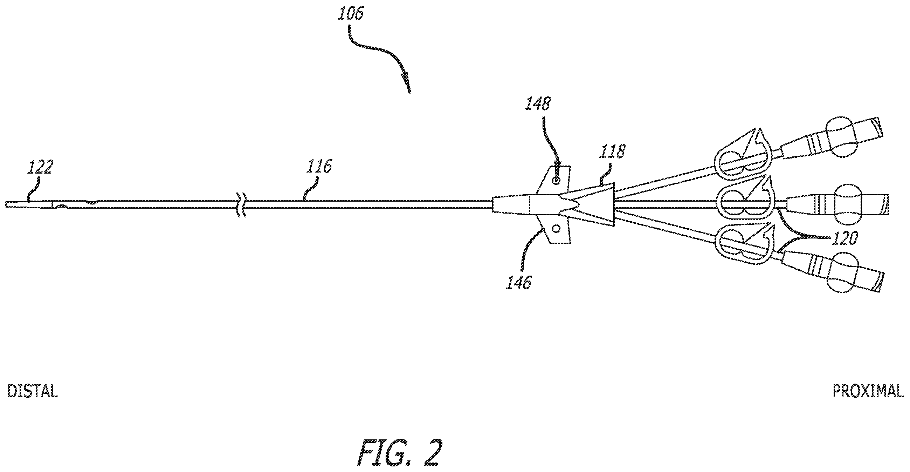

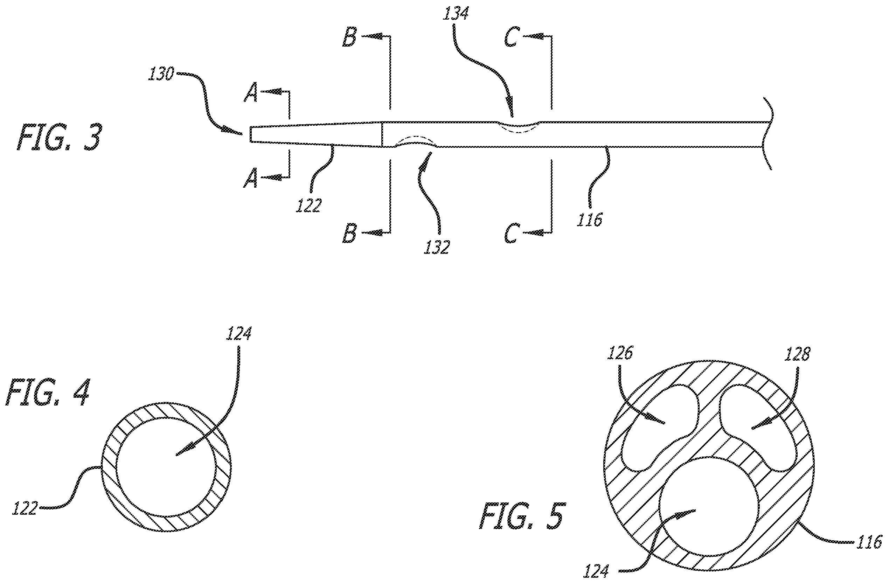

FIG. 2 provides the RICC 106 in accordance with some embodiments. FIG. 3 provides a distal portion of the RICC 106, FIG. 4 provides a first transverse cross section of the distal portion of the RICC 106, and FIG. 5 provides a second or third transverse cross section of the distal portion of the RICC 106 in accordance with some embodiments.

As shown, the RICC 106 can include a catheter tube 116, a catheter hub 118, and one or more extension legs 120.

The catheter tube 116 can include a catheter tip 122 in a distal portion of the catheter tube 116 and one or more catheter-tube lumens through the catheter tube 116.

The one-or-more catheter-tube lumens can extend through an entirety of the catheter tube 116; however, only one catheter-tube lumen typically extends from a proximal end of the catheter tube 116 to a distal end of the catheter tube 116 in a multiluminal RICC (e.g., a diluminal RICC, a triluminal RICC, a tetraluminal RICC, a pentaluminal RICC, a hexaluminal RICC, etc.). *(See* FIGS. 3-5.) Indeed, the catheter tip 122, typically includes a single lumen therethrough, whether or not the catheter tip 122 is formed integrally with the catheter tube 116 or separately from the catheter tube 116 and coupled thereto.

The catheter hub 118 can be coupled to a proximal portion of the catheter tube 116. The catheter hub 118 can include one or more catheter-hub lumens corresponding in number to the one-or-more catheter-tube lumens. The one-or-more catheter-hub lumens can extend through an entirety of the catheter hub 118 from a proximal end of the catheter hub 118 to a distal end of the catheter hub 118.

Each extension leg of the one-or-more extension legs 120 can be coupled to the catheter hub 118 by a distal portion thereof. The one-or-more extension legs 120 can respectively include one or more extension-leg lumens, which, in turn, correspond in number to the one-or-more catheter-hub lumens. Each extension-leg lumen of the one-or-more extension-leg lumens can extend through an entirety of the extension leg from a proximal end of the extension leg to a distal end of the extension leg.

Each extension leg of the one-or-more extension legs 120 can include a Luer connector coupled to the extension leg, through which Luer connector the extension leg and the extension-leg lumen thereof can be connected to another medical device and a lumen thereof.

As shown, the RICC 106 can be a triluminal RICC including a set of three lumens. The set of three lumens can include a primary lumen 124, a secondary lumen 126, and a tertiary lumen 128 formed of fluidly connected portions of three catheter-tube lumens, three catheter-hub lumens, and three extension-leg lumens. The primary lumen 124 can have a primary-lumen aperture 130 in a distal end of the catheter tip 122, the distal end of the catheter tube 116, or a distal end of the RICC 106. The secondary lumen 126 can have a secondary-lumen aperture 132 in a side of the distal portion of the catheter tube 116. The tertiary lumen 128 can have a tertiary-lumen aperture 134 in the side of the distal portion of the catheter tube 116 proximal of the secondary-lumen aperture 132.

The access guidewire 112 can be disposed in the primary lumen 124 of the RICC 106 in the RICC assembly 108. The access guidewire 112 can include a distal portion extending from the distal end of the RICC 106 as evidenced by comparison of at least FIGS. 7 and 8. The access guidewire 112 can also include a proximal portion extending from a proximal end of the RICC 106 as shown in FIG. 6. As set forth below, the proximal portion of the access guidewire 112 extending from the proximal end of the RICC 106 or a guidewire hub 135 coupled thereto can be attached to the guidewire attachment point 196 in the closed end of the enclosure 192 in the initial position of the RICC assembly 108 in the frame 172.

The keeper 114 can include a splittable casing 136 and a catheter-hub holder 138 to which a proximal end of the splittable casing 136 is attached.

FIGS. 7 and 8 provide transverse cross sections of the RICC assembly 108, which, in turn, provide the splittable casing 136 as part of a longitudinal composite 140 of the RICC assembly 108 in accordance with some embodiments.

As shown, the splittable casing 136 can form the longitudinal composite 140 with the catheter tube 116, the access guidewire 112, or both the catheter tube 116 and the access guidewire 112 in the RICC assembly 108 depending upon the embodiment of the RICC assembly 108 and portion thereof. With respect to the RICC assembly 108 of FIG. 6, for example, the splittable casing 136 can be over both the catheter tube 116 and the access guidewire 112 in a proximal portion of the RICC assembly 108 as shown in FIG. 7. The splittable casing 136 can also be over the distal portion of the access guidewire 112 extending from the distal end of the RICC 106 as shown in FIG. 8. The splittable casing 136 is configured to split along its length such that the distal portion of the access guidewire 112 is initially revealed and the distal portion of the catheter tube 116 is subsequently revealed. In this way, the splittable casing 136 is configured to keep the catheter tube 116 and at least the distal portion of the access guidewire 112 sterile until deployed.

The catheter-hub holder 138 is configured to hold the catheter hub 118 therein as well as keep the splittable casing 136 in position over the catheter tube 116 and the access guidewire 112. The catheter-hub holder 138 includes a perimetrical wall 142 around at least a portion (e.g., a proximal portion) of a perimeter of the catheter-hub holder 138. The perimetrical wall 142 defines a recess into which the catheter hub 118 fits with an engineering fit (e.g., a clearance fit such as a running, sliding, or location fit or a transition fit such as a similar or fixed fit as classified by the International Organization for Standardization ["ISO"]) as well as one or more gaps 144 for extension of the one-or-more extension legs 120 therethrough. Additionally or alternatively, the catheter-hub holder 138 can include a wing corresponding to a suture wing 146 of the catheter hub 118. Such a wing can include posts configured to insert into suture-wing holes 148 of the suture wing 146 of the catheter hub 118 with an engineering fit.

FIG. 9 provides a first alternative RICC assembly 150 in accordance with some embodiments. FIG. 10 provides a transverse cross section of the RICC assembly 150 in accordance with some embodiments.

As shown, the RICC assembly 150 can include the RICC 106, the access guidewire 112, and the keeper 114; however, the splittable casing 136 over the catheter tube 116 and the access guidewire 112 can have a larger diameter over the catheter tube 116 and a smaller diameter over the access guidewire 112, thereby more closely following contours of the catheter tube 116 and the access guidewire 112. Advantageously, the splittable casing 136 can keep the catheter tube 116 from prematurely advancing over the access guidewire 112 while advancing the RICC assembly 150 from the initial position in the frame 172 to the final position in the frame 172 as set forth below.

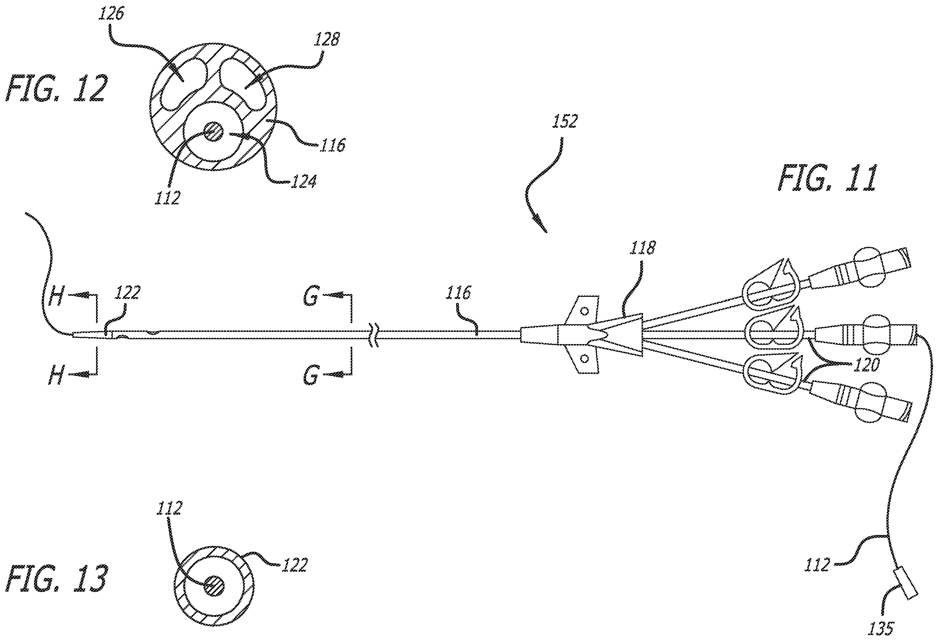

FIG. 11 provides a second alternative RICC assembly 152 in accordance with some embodiments. FIG. 12 provides a first transverse cross section of the RICC assembly 152 in accordance with some embodiments. FIG. 13 provides a second transverse cross section of the RICC assembly 152 in accordance with some embodiments.

As shown, the RICC assembly 152 can include the RICC 106 and the access guidewire 112 without the keeper 114 and the splittable casing 136 thereof. Optionally, the catheter tube 116 and the access guidewire 112 can be disposed in a clear covering such as a collapsible plastic bag configured to keep the catheter tube 116 and the distal portion of the access guidewire 112 sterile until deployed. Whether the catheter tube 116 and the access guidewire 112 are bare or disposed in the covering, a transition between the distal portion of the access guidewire 112 and the catheter tube 116 can be advantageously visualized while advancing the RICC assembly 152 from the initial position in the frame 172 to the final position in the frame 172 as set forth below.

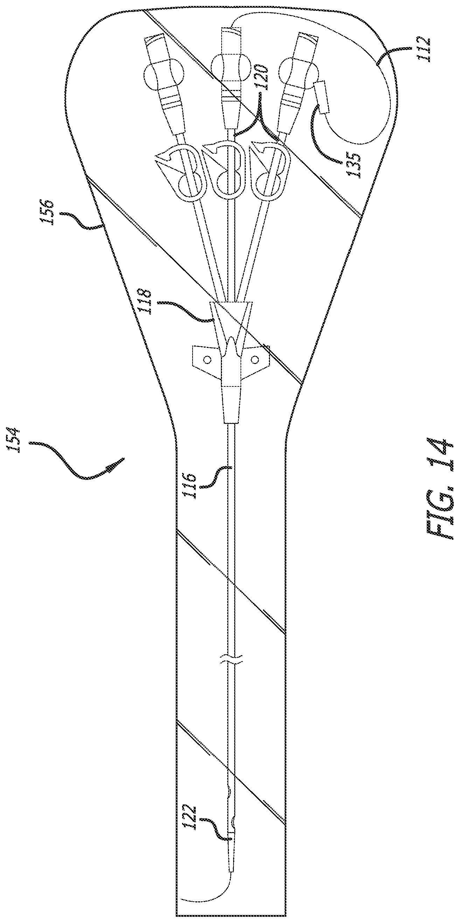

FIG. 14 provides a third alternative RICC assembly 154 in accordance with some embodiments.

As shown, the RICC assembly 154 can include the RICC 106 and the access guidewire 112 without the keeper 114 and the splittable casing 136 thereof. Instead of the optional covering over the catheter tube 116 and the access guidewire 112 of the RICC assembly 152, an entirety of the RICC assembly 154 can be disposed a clear covering 156 such as a collapsible or zippered plastic bag configured to keep the catheter tube 116 and the distal portion of the access guidewire 112 sterile until deployed. Like the RICC assembly 152, the transition between the distal portion of the access guidewire 112 and the catheter tube 116 can be advantageously visualized through the covering 156 while advancing the RICC assembly 154 from the initial position in the frame 172 to the final position in the frame 172 as set forth below.

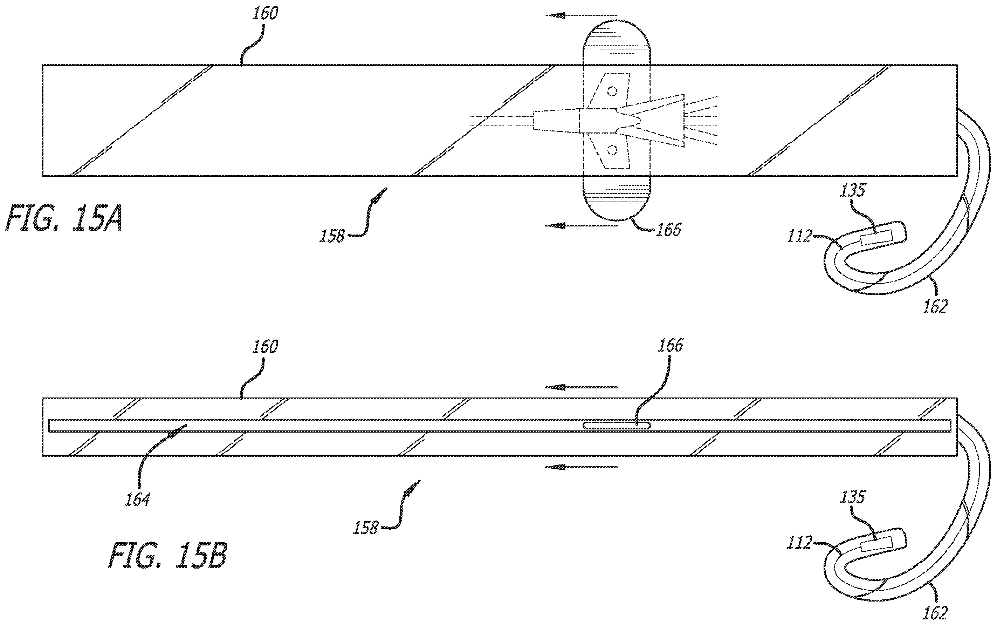

FIGS. 15A and 15B provide different views of a fourth alternative RICC assembly 158 in accordance with some embodiments.

As shown, the RICC assembly 158 can include the RICC 106 and the access guidewire 112 without the keeper 114 and the splittable casing 136 thereof; however, the RICC assembly 158 including the catheter tube 116 and the distal portion of the access guidewire 112 can be disposed in a longitudinal housing 160 with the proximal portion of the access guidewire 112 extending therefrom but disposed in a clear covering 162 such as a collapsible plastic bag. The housing 160 can include longitudinal slots 164 configured for advancing or withdrawing a RICC-advancement wing 166 therein. The RICC-advancement wing 166 can be configured like the catheter-hub holder 138 such that the catheter hub 118 fits in the RICC-advancement wing 166 with an engineering fit or the suture wing 146 thereof fits over posts of the RICC-advancement wing 166. Regardless, the housing 160 can be configured to keep the catheter tube 116 and the distal portion of the access guidewire 112 sterile until deployed. Advantageously, the housing 160 can prevent the guidewire hub 135 of the access guidewire 112 from passing into the housing 160, thereby controlling relative movement of the catheter tube 116 and the access guidewire 112.

### RICC insertion devices

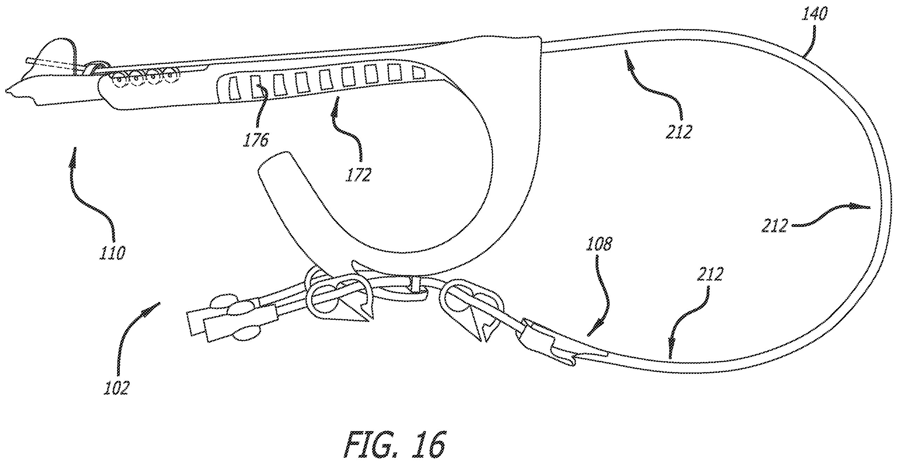

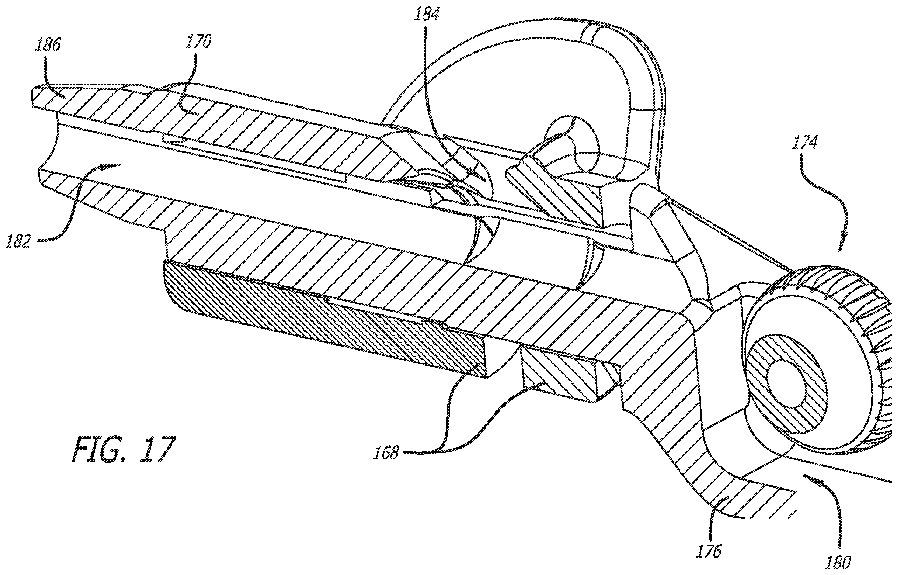

FIG. 16 provides the RICC insertion assembly 102 including the RICC assembly 108 disposed in the RICC insertion device 110 in accordance with some embodiments. FIG. 17 provides a nose cover 168 over a nose 170 of a frame 172 of the RICC insertion device 110 in accordance with some embodiments.

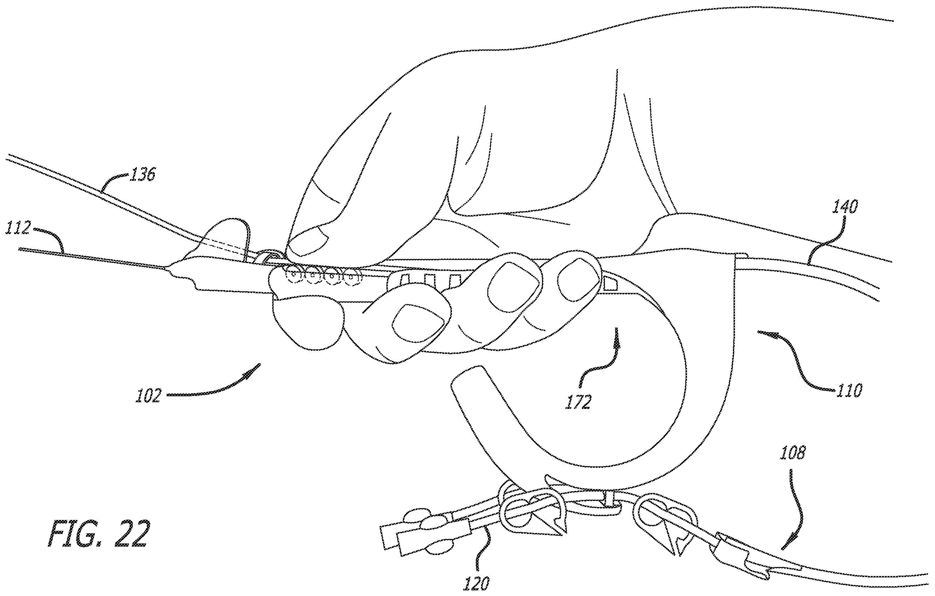

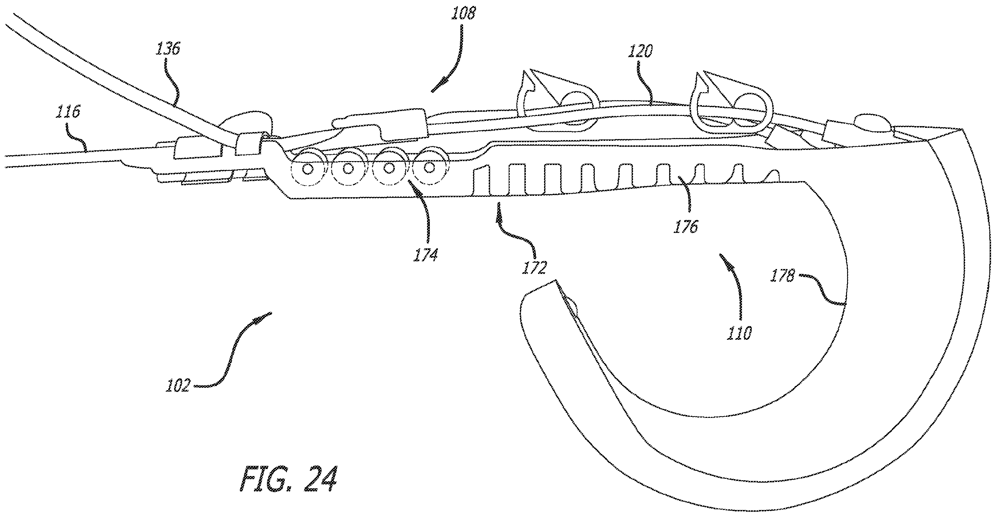

As shown, the RICC insertion device 110 can include the frame 172, one or more roller wheels 174, and the nose cover 168 over the nose 170 of the frame 172. The RICC insertion device 110 can be configured for advancing the RICC assembly 108 from an initial position in the frame 172, (*see* FIG. 16) through one or more intermediate positions in the frame *172, (see* FIG. 22) to a final position in the frame 172 (*see* FIG. 24) for insertion of the access guidewire 112 and the catheter tube 116 of the RICC 106 into a blood-vessel lumen of a patient subsequent to establishment of a needle tract to the blood-vessel lumen.

The frame 172 can include a longitudinal handle 176, the nose 170, and a curved cradle 178; however, in some embodiments, the cradle 178 can be a reel.

The handle 176 can include a wheel well 180 in a distal portion of the handle 176. The wheel well 180, in turn, can include the one-or-more roller wheels 174 disposed therein. The handle 176 can be configured for holding and operating the RICC insertion device 110 with a single hand (e.g., a left or right hand). For example, when the RICC assembly 108 is disposed in the RICC insertion device 110, the RICC assembly 108 can be advanced from the initial position in the frame 172, through the one-or-more intermediate positions in the frame 172, to the final position in the frame 172 with a single hand by repeatedly pushing the longitudinal composite 140 of the RICC assembly 108 into the one-or-more roller wheels 174 and rolling the longitudinal composite 140 across the one-or-more roller wheels 174 with a thumb of the hand while cradling the handle 176 of the RICC insertion device 110 in fingers of the same hand.

The nose 170 can be integral with the handle 176, extending from the distal portion of the handle 176 distal of the wheel well 180. The nose 170 can include a through channel 182 configured for advancing the access guidewire 112 and the catheter tube 116 of the RICC assembly 108 therethrough. Together, the nose 170 and the nose cover 168 thereover can form a split channel 184 configured for both splitting and passing the splittable casing 136 of the RICC assembly 108 therethrough. Advantageously, the nose 170 can also include a male nose connector 186 configured to be disposed in the female valved-cap connector 232 of the sheath hub 226 of the introducer sheath 216 set forth below, which facilitates advancing the access guidewire 112 through the introducer sheath.

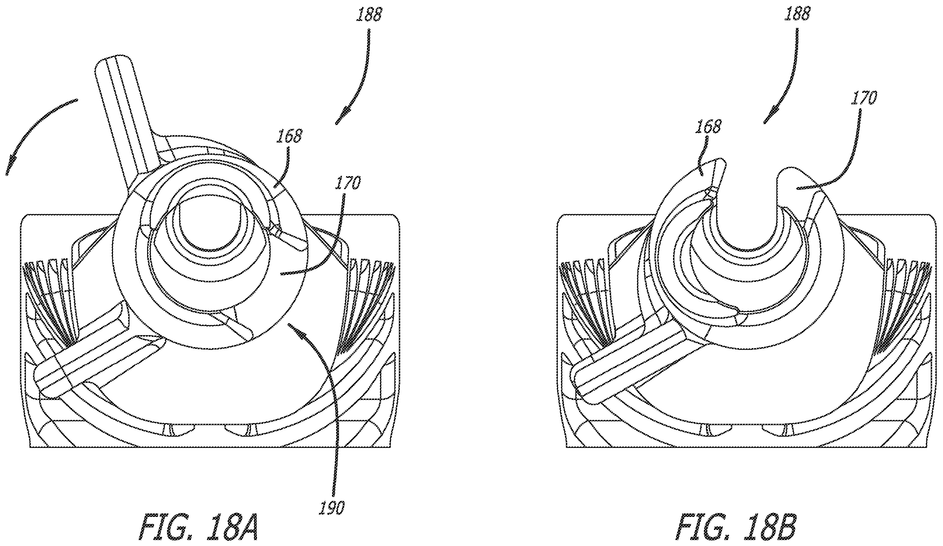

FIG. 18A and FIG. 18B provide a retaining clamp 188 formed between the nose 170 and the nose cover 168 with the retaining clamp 188 respectively in a closed state and an open state in accordance with some embodiments.

As shown, the nose 170 and the nose cover 168 thereover can also form the retaining clamp 188. The retaining clamp 188 can be configured to slidably clamp therein the longitudinal composite 140 of the RICC assembly 108 or the splittable casing 136, the catheter tube 116, or the access guidewire 112 thereof. The nose cover 168 can be rotatable over the nose 170. In addition, the nose cover 168 can include a longitudinal gap 190 configured to rotatably align with the through channel 182 in the nose 170 for removal of the longitudinal composite 140 of the RICC assembly 108 or the splittable casing 136, the catheter tube 116, or the access guidewire 112 therefrom. Indeed, if the longitudinal composite 140 is partially or fully split, the longitudinal gap 190 of the nose cover 168 can be rotatably aligned with the through channel 182 in the nose 170 for removal of the splittable casing 136, the catheter tube 116, or the access guidewire 112 therefrom.

The cradle 178 can be integral with the handle 176, extending from a proximal portion of the handle 176 and curving back toward the distal portion of the handle 176. The cradle 178 can include an open face, an enclosure 192 over a distal portion of the cradle 178, and a retaining arch 194 over the cradle 178 proximate the proximal portion of the handle 176 from which the cradle 178 extends.

The open face of the cradle 178 can be configured for cradling therein the RICC assembly 108 (e.g., the longitudinal composite 140, the catheter hub 118, the one-or-more extension legs 120, etc.) from the initial position of the RICC assembly 108 in the frame 172, through the one-or-more intermediate positions of the RICC assembly 108 in the frame 172, to the final position of the RICC assembly 108 in the frame 172.

The enclosure 192 over the cradle 178 can be configured to enclose therein the otherwise exposed proximal portion of the access guidewire 112 as well the extension leg of the one-or-more extension legs 120 from which the proximal portion of the access guidewire 112 extends in the initial position of the RICC assembly 108 in the frame 172. *(See* FIGS. 16, 22, and 23A.) Indeed, the foregoing proximal portion of the access guidewire 112 or the guidewire hub 135 coupled thereto can be attached to a guidewire attachment point 196 in a closed end of the enclosure 192 in the initial position of the RICC assembly 108 in the frame 172. In this way, the enclosure 192 can keep at least the proximal portion of the access guidewire 112 extending from the proximal end of the RICC 106 touch-free and sterile.

The retaining arch 194 over the cradle 178 is configured to retain the RICC assembly 108 in the RICC insertion device 110. The retaining arch 194 is also configured to hold the longitudinal composite 140 over the handle 176 as the RICC assembly 108 transitions over the proximal portion of the handle 176 from which the cradle 178 extends upon advancing the RICC assembly 108 from the initial position to the final position in the frame 172.

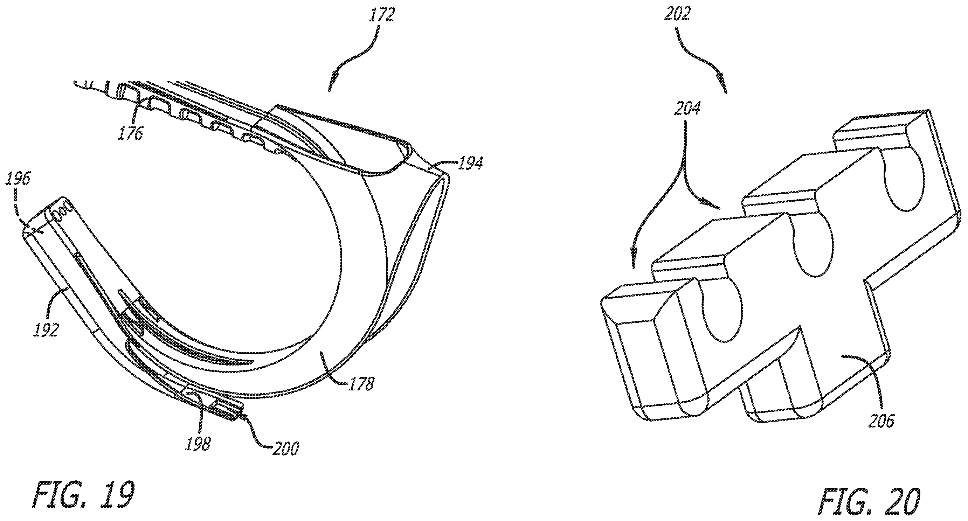

FIG. 19 provides a curved extension 198 of the cradle 178 extending from the enclosure 192 in accordance with some embodiments.

As shown, the extension 198 can extend from an open end of the enclosure 192 over the distal portion of the cradle 178 substantially following a same curve as the cradle 178. The extension 198 can include a slot 200 in the extension 198 that faces the open face of the cradle 178. As set forth below, the slot 200 can be configured to engage with the retaining clip 202.

FIG. 20 provides a retaining clip 202 of the RICC insertion device 110 in accordance with some embodiments.

As shown, the retaining clip 202 can be a separate piece of the RICC insertion device 110 including one or more extension-leg clips 204 and a post 206. The retaining clip 202 can be configured to clip on to the proximal portion of the RICC 106 and retain the RICC assembly 108 in the RICC insertion device 110. More specifically, the retaining clip 202 can be configured to retain the RICC 106, itself, in position in the RICC insertion device 110, which, in turn, can help keep the RICC 106 and the catheter tube 116 thereof from prematurely advancing over the access guidewire 112 when advancing the RICC assembly 108 in the frame 172.

The one-or-more extension-leg clips 204 can be configured to clip on to the one-or-more extension legs 120. While the one-or-more extension-leg clips 204 can include a dedicated extension-leg clip for each extension leg of the one-or-more extension legs 120 as shown, the one-or-more extension-leg clips 204 can alternatively include a single extension-leg clip configured to clip on to any single extension leg of two or more of the extension legs 120, a pair of the extension-leg clips 204 configured to clip on to any two extension legs of three or more of the extension legs 120, etc.

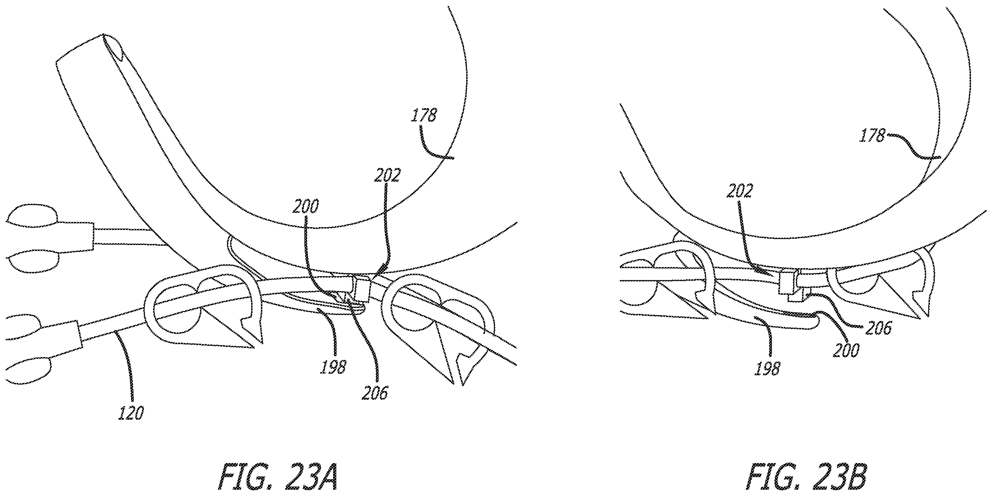

The post 206 can be configured to engage with the slot 200 in the extension 198 of the cradle 178 in the initial position of the RICC assembly 108 in the frame 172 as well as through some earlier intermediate positions of the one-or-more intermediate positions of the RICC assembly 108 in the frame 172. *(See* FIGS. 16, 22, and 23A.) However, the retaining clip 202 can be configured to disengage with the slot 200 in the extension 198 when any remaining slack of the slack loop 212 of the longitudinal composite 140 is removed in the RICC insertion assembly 102 upon advancing the RICC assembly 108 through a transition to a later intermediate position of the one-or-more intermediate positions of the RICC assembly 108 in the frame 172. *(See* FIG. 23B.) The retaining clip 202 can be configured to remain clipped on the one-or-more extension legs 120 through the transition and follow the cradle 178 over the proximal portion of the handle 176 to a medial portion of the handle 176 in the final position of the RICC assembly 108 in the frame 172. *(See* FIG. 24.)

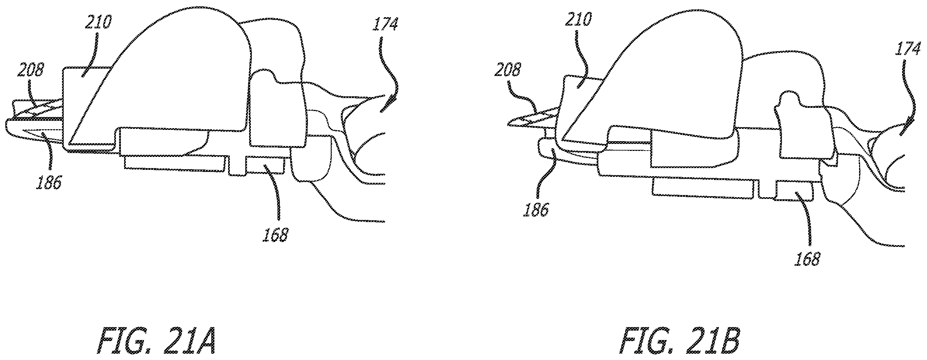

FIG. 21A and 21B provide a retractable nicking blade 208 integrated into the nose 170 of the RICC insertion device 110 with the nicking blade 208 respectively in a safety position and a nicking position in accordance with some embodiments.

As shown, the nicking blade 208 can be disposed or overmolded in a nicking-blade carriage 210 slidably integrated into the nose 170. When the nicking-blade carriage 210 is in the safety position, the nicking blade 208 can be short of a distal end of the nose 170 to prevent inadvertent nicking; however, when the nicking-blade carriage 210 is in the nicking position, the nicking blade 208 can extend beyond the distal end of the nose 170 for nicking an area of skin. Notably, the nicking blade 208 can be configured for nicking the area of skin around a needle tract for subsequent insertion of the catheter tube 116 when the catheter tube 116 is 7 Fr or larger.

### RICC insertion assemblies

Again, FIG. 16 provides the RICC insertion assembly 102 including the RICC assembly 108 disposed in the initial position in the frame 172 of the RICC insertion device 110, thereby forming the ready-to-operate state of the RICC insertion assembly 102 in accordance with some embodiments.

The RICC insertion assembly 102, which includes the RICC assembly 108 disposed in the RICC insertion device 110, can be configured for advancing the RICC assembly 108 from the initial position in the frame 172 of the RICC insertion device 110, through the one-or-more intermediate positions in the frame 172, to the final position in the frame 172 for insertion of the access guidewire 112 and the catheter tube 116 of the RICC 106 into a blood-vessel lumen of a patient subsequent to establishment of a needle tract to the blood-vessel lumen.

In the initial position of the RICC assembly 108 in the frame 172 of the RICC insertion device 110, a distal end of the longitudinal composite 140 can be substantially commensurate with a distal end of the frame 172 (e.g., the nose 170) if the longitudinal composite 140 is not split. If the longitudinal composite 140 is partially split as shown in FIG. 16, a distal end of the splittable casing 136 or the distal end of the access guidewire 112 can be substantially commensurate with the distal end of the frame 172 in the initial position of the RICC assembly 108 in the frame 172. Regardless, the retaining clamp 188 of the RICC insertion device 110 can clamp therein a distal portion of the longitudinal composite 140 in the initial position of the RICC assembly 108 in the frame 172. Further in the initial position of the RICC assembly 108 in the frame 172, a proximal end of an extension leg of the one-or-more extension legs 120 or a Luer connector coupled to the extension leg can be substantially commensurate with a proximal end of the cradle 178 of the RICC insertion device 110. In addition, a proximal end of the access guidewire 112 or the guidewire hub 135 thereof can be coupled to the proximal end of the cradle 178 (e.g., the guidewire attachment point 196 in the closed end of the enclosure 192) in the initial position of the RICC assembly 108 in the frame 172. As a result of clamping the distal portion of the longitudinal composite 140 with the retaining clamp 188 and attaching the proximal end of the access guidewire 112 or the guidewire hub 135 thereof to the proximal end of the cradle 178, a slack loop 212 of the longitudinal composite 140 can extend away from both the handle 176 and the cradle 178 in the initial position of the RICC assembly 108 in the frame 172.

FIG. 22 provides the RICC insertion assembly 102 with the RICC assembly 108 in an earlier intermediate position in the frame 172 of the RICC insertion device 110 in accordance with some embodiments. FIG. 23A provides the RICC insertion assembly 102 with the RICC assembly 108 in a same or different earlier intermediate position in the frame 172 than FIG. 22 in which the slack loop 212 of the longitudinal composite 140 remains in the RICC insertion assembly 102 in accordance with some embodiments. FIG. 23B provides the RICC insertion assembly 102 with the RICC assembly 108 in a later intermediate position in the frame 172 in which no slack loop of the longitudinal composite 140 remains in the RICC insertion assembly 102 in accordance with some embodiments.

As shown, in an earlier intermediate position of the RICC assembly 108 in the frame 172 of the RICC insertion device 110, the slack loop 212 of the longitudinal composite 140 can remain in the RICC insertion assembly 102 and extend away from both the handle 176 and the cradle 178 of the RICC insertion device 110. In addition, the post 206 of the retaining clip 202 can remain engaged with the slot 200 in the extension 198 over the cradle 178, thereby retaining the RICC 106, itself, in position in the RICC insertion device 110 such that the catheter tube 116 cannot prematurely advance over the access guidewire 112 when advancing the RICC assembly 108 in the frame 172. However, the retaining clip 202 can be configured to disengage with the slot 200 in the extension 198 when any remaining slack of the longitudinal composite 140 is removed in the RICC insertion assembly 102 upon advancing the RICC assembly 108 through the transition to a later intermediate position. Indeed, when the remaining slack of the longitudinal composite 140 is removed from the RICC insertion assembly 102, the post 206 of the retaining clip 202 can disengage with the slot 200 in the extension 198 such as by being pulled out of the slot 200, thereby allowing the RICC 106 to advance over the access guidewire 112 with advancement of the longitudinal composite 140. In the later intermediate position of the RICC assembly 108 in the frame 172, no slack loop of the longitudinal composite 140 remains in the RICC insertion assembly 102, thereby allowing the RICC 106 to continue to advance over the access guidewire 112 with advancement of the longitudinal composite 140.

FIG. 24 provides the RICC insertion assembly 102 with the RICC assembly 108 in the final position in the frame 172 of the RICC insertion device 110 assembly in accordance with some embodiments.

As shown, in the final position of the RICC assembly 108 in the frame 172 of the RICC insertion device 110, the longitudinal composite 140 can be substantially split, the catheter hub 118 of the RICC 106 can lie over the wheel well 180 of the frame 172, and the one-or-more extension legs 120 of the RICC 106 can lie substantially over the handle 176 of the frame 172.

### Introducers

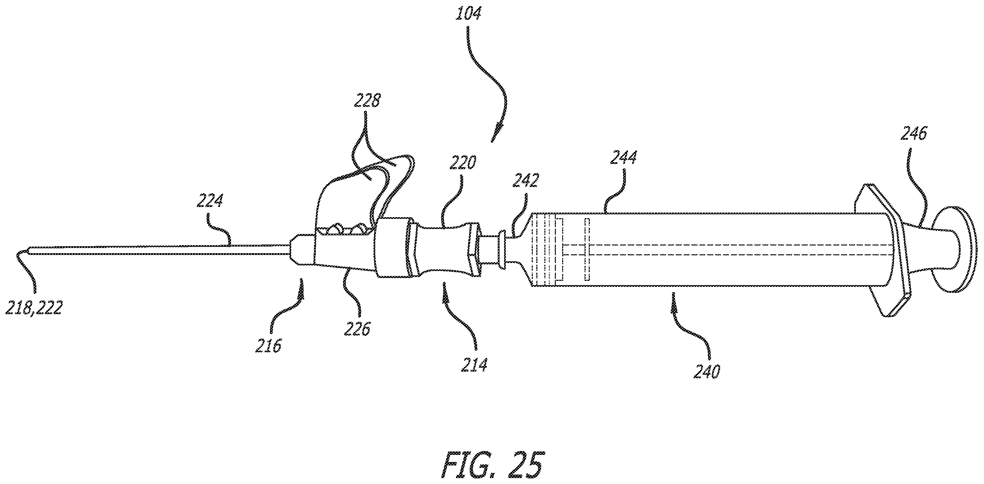

FIG. 25 provides the introducer 104 in accordance with some embodiments.

As shown, the introducer 104 can include an introducer needle 214 and a splittable introducer sheath 216. The introducer sheath 216 can be configured to accept therein the introducer needle 214. Indeed, the introducer sheath 216 can be disposed over the introducer needle 214 in a ready-to-operate state of the introducer 104 as shown in FIG. 25. Advantageously, the introducer 104 in combination with the RICC insertion assembly 102 can save a clinician's time in that the introducer needle 214 does not have to be pulled out over the access guidewire 112 and the catheter tube 116 does not have to be threaded over the access guidewire 112.

The introducer needle 214 can include a needle shaft 218 and a needle hub 220. The needle shaft 218 can include a needle tip 222 in a distal portion of the needle shaft 218 configured for establishing a needle tract from an area of skin into a blood-vessel lumen. The needle hub 220 can be coupled to a proximal portion of the needle shaft 218. Advantageously, the introducer needle 214 can be a smaller size than needles typically used to place CVCs, which makes blood-vessel access more convenient. Consequently, the introducer sheath 216 can be configured to not accept therein the catheter tube 116 of the RICC 106.

The introducer sheath 216 can include a splittable sheath body 224 and a splittable sheath hub 226 coupled to a proximal portion of the sheath body 224. Advantageously, the introducer sheath 216 can be configured to split from end to end along a side or a pair of opposing sides of the introducer sheath 216.

The sheath body 224 can be formed of polytetrafluoroethylene, which facilitates smooth, consistent splitting without faults in a side or a pair of opposing sides of the sheath body 224. That said, the sheath body 224 can be formed of any of a number of other polymers (e.g., polyethylene); however, the sheath body 224 can generally benefit by including a single longitudinal fault (e.g., a score, a perforation, etc.) along the side of the sheath body 224 hub or a pair of longitudinal faults including a primary fault and a secondary fault along the pair of opposing sides of the sheath body 224 when formed of the other polymers.

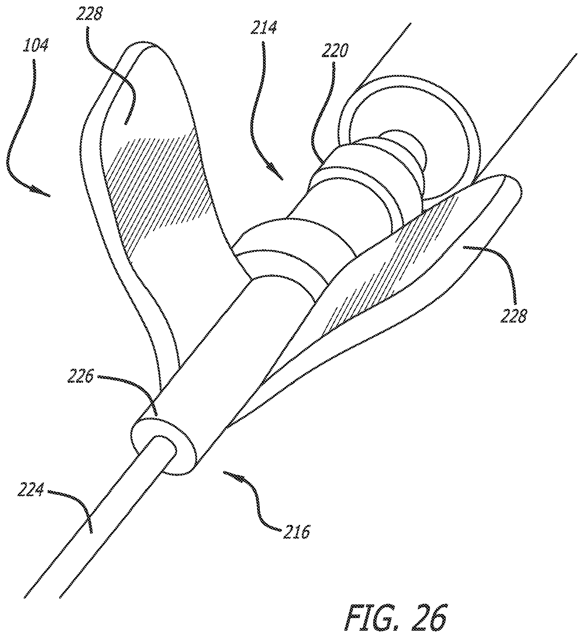

FIG. 26 provides a pair of outwardly extending wings 328 along a length of the sheath hub 226 in accordance with some embodiments.

As shown, the sheath hub 226 can include the wings 328 along a length of the sheath hub 226. The wings 328 can have an internal angle of about 90° or less between the wings 328 configured for splitting the sheath hub 226 by pinching the wings 328 together with a single hand.

The sheath hub 226 can include a single longitudinal fault along a side of the sheath hub 226 or a pair of longitudinal faults including a primary fault and a secondary fault along a pair of opposing sides of the sheath hub 226. When the sheath hub 226 includes the single longitudinal fault, the single longitudinal fault can be along the side of the sheath hub 226 opposite a vertex of the internal angle formed by the wings 328 such that the vertex of the internal angel can function as a living hinge. The sheath hub 226 can be configured to split along the fault for propagation along a same side of the sheath body 224 when the wings 328 are pinched together. When the sheath hub 226 includes the pair of longitudinal faults, the primary fault can extend along a primary side of the sheath hub 226 opposite the vertex of the internal angle formed by the wings 328 and the secondary fault can extend along a secondary side of the sheath hub 226 opposite the primary fault. The sheath hub 226 can be configured to split along the primary fault for propagation along the primary side of the sheath body 224 when the wings 328 are pinched together. The sheath hub 226 can also be configured to split along the secondary fault for propagation along the secondary side of the sheath body 224 when the wings 328 are pulled apart.

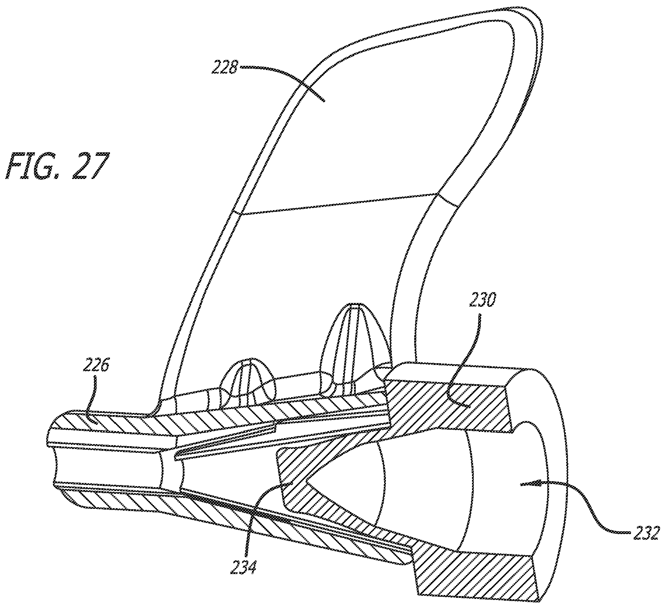

FIG. 27 provides a longitudinal cross section of the sheath hub 226 including a valved cap 230 in accordance with some embodiments.

As shown, the sheath hub 226 can include the valved cap 230 disposed in a proximal portion of the sheath hub 226. The valved cap 230 can include a tapered female valved-cap connector 232 and a septum 234 distal of a proximal opening in the female valved-cap connector 232. The female valved-cap connector 232 can be configured to accept therein a tapered male needle-hub connector extending from a distal portion of the needle hub 220. The septum 234 can be configured to accept therethrough the needle shaft 218. Indeed, the male needle-hub connector can be disposed in the female valved-cap connector 232 in the ready-to-operate state of the introducer 104. In addition, the needle shaft 218 can pass through the septum 234 such that the needle tip 222 extends beyond a distal end of the sheath body 224 in the ready-to-operate state of the introducer 104. Notably, the valved cap 230 can be partially or fully split such that the valved cap 230 splits with the sheath hub 226 when the wings 328 are pinched together.

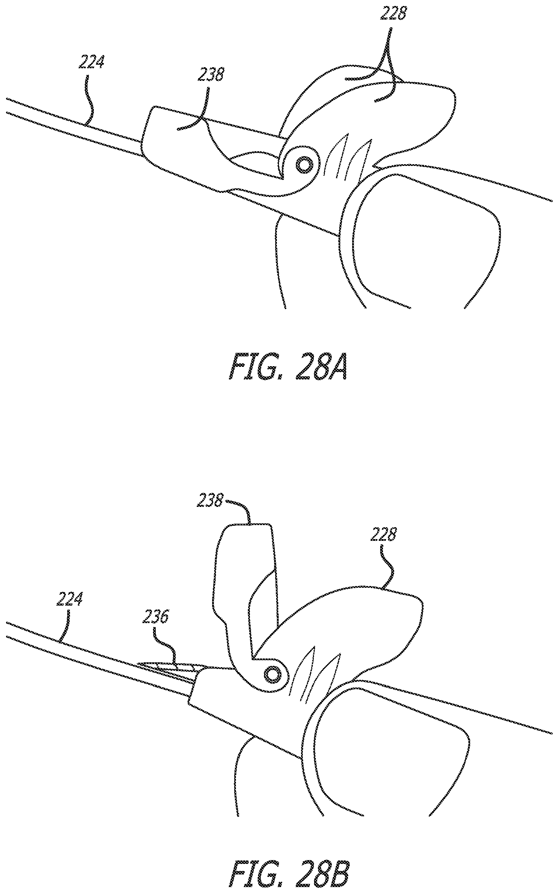

FIGS. 28A and 28B provide an overmolded nicking blade 236 integrated into the sheath hub 226 with a hinged nicking-blade cover 238 respectively in a closed state or safety position and an opened state or nicking position in accordance with some embodiments.

As shown, the sheath hub 226 can include the overmolded nicking blade 236 and the nicking-blade cover 238. The nicking blade 236 can distally extend from the sheath hub 226. When the nicking-blade cover 238 is in the closed state or safety position, the nicking-blade cover 238 can be closed over the nicking blade 236 to prevent inadvertent nicking; however, when the nicking-blade cover 238 is in the opened state or nicking position, the nicking-blade cover 238 can be open away from the nicking blade 236 for nicking an area of skin.

The introducer 104 can further include a syringe 240. The syringe 240 can include a tapered male syringe tip 242 extending from a distal portion of the syringe 240 configured to insert into a tapered female needle-hub connector in a proximal portion of the needle hub 220. The syringe 240 can be configured with a barrel 244 and a plunger 246 to aspirate blood for confirmation of blood-vessel access upon establishing a needle tract from an area of skin into a blood-vessel lumen.

### Methods (which are not part of the invention)

A method of the RICC insertion system 100 includes a method for inserting the RICC 106 into a blood-vessel lumen of a patient, which is not part of the invention, Such a method includes, in some embodiments, one or more steps selected from an obtaining step, a needle tract-establishing step, a blood-aspirating step, an introducer sheath-advancing step, an introducer needle-removing step, a connector-connecting step, a skin-nicking step, an access guidewire-advancing step, an introducer sheath-removing step, a catheter tube-advancing step, an access guidewire-removing step, a keeper-removing step, a catheter tube-freeing step, a maneuver guidewire-inserting step, a catheter tube-placing step, and a maneuver guidewire-withdrawing step.

The obtaining step can include obtaining the RICC insertion system 100. As set forth above, the RICC insertion system 100 can include the introducer 104, the RICC insertion device 110, and the RICC assembly 108 including the RICC 106. The RICC assembly 108 can be already disposed in the RICC insertion device 110 in the substantially ready-to-operate state of the RICC insertion assembly 102.

The needle tract-establishing step can include establishing a needle tract from an area of skin into the blood-vessel lumen of the patient with the introducer needle 214 of the introducer 104. In addition, the needle tract-establishing step can include ensuring blood flashes back into the needle hub 220 of the introducer needle 214 for confirmation the needle tract extends into the blood-vessel lumen. The introducer needle 214 can be disposed in the introducer sheath 216 of the introducer 104 for the needle tract-establishing step.

The blood-aspirating step can include aspirating blood with the syringe 240 coupled to the introducer needle 214 for confirmation the needle tract extends into the blood-vessel lumen. The blood-aspirating step can be performed before the introducer needle-removing step.

The introducer sheath-advancing step can include advancing the introducer sheath 216 over the introducer needle 214 and farther into the blood-vessel lumen. The introducer sheath-advancing step can be performed before the introducer needle-removing step.

The introducer needle-removing step can include removing the introducer needle 214 from the introducer sheath 216 leaving the introducer sheath 216 in the blood-vessel lumen, thereby allowing the access guidewire 112 to be inserted into the introducer sheath 216.

The connector-connecting step can include inserting the male nose connector 186 of the nose 170 of the RICC insertion device 110 into the female valved-cap connector 232 of the sheath hub 226 of the introducer sheath 216. The connector-connecting step can be performed before the access guidewire-advancing step.

The skin-nicking step can include nicking the area of skin around the needle tract with the nicking blade 236 extending from the sheath hub 226. The skin-nicking step can be performed when the catheter tube 116 is 7 Fr or larger. The skin-nicking step can be accompanied by a cover-opening step before the skin-nicking step and a cover-closing step after the skin-nicking step. The cover-opening step can include opening the nicking-blade cover 238 away from the nicking blade 236 and into the nicking position away from the nicking blade 236. The cover-closing step can include closing the nicking-blade cover 238 over the nicking blade 236 into the safety position over the nicking blade 236. The skin-nicking step can be performed before the access guidewire-advancing step.

The access guidewire-advancing step can include advancing the access guidewire 112 through the nose 170 and nose cover 168 of the RICC insertion device 110, through the sheath hub 226 of the introducer 104, through the introducer sheath 216, and into the blood-vessel lumen. The access guidewire-advancing step can be accomplished, in part, by splitting the splittable casing 136 away from the longitudinal composite 140 of the RICC assembly 108. The distal portion of the access guidewire 112 that extends from the primary-lumen aperture 130 of the catheter tube 116 of the RICC 106 can be freed from the longitudinal composite 140 with the splitting of the splittable casing 136 for the access guidewire-advancing step.

The introducer sheath-removing step can include splitting the introducer sheath 216 to form a split along a side of the introducer sheath 216 by pinching together the wings 328 outwardly extending from the sheath hub 226. The introducer sheath-removing step can also include propagating the split in a side of the sheath hub 226 along a same side of the sheath body 224 of the introducer 104. Ultimately, the introducer sheath-removing step can include removing the split introducer sheath leaving the access guidewire 112 in the blood-vessel lumen.

The skin-nicking step can alternatively include nicking the area of skin around the needle tract with the retractable nicking blade 208 integrated into the nose 170 of the RICC insertion device 110. Such a skin-nicking step is also performed when the catheter tube 116 is 7 Fr or larger. The skin-nicking step can be accompanied by a pair of carriage-sliding steps including a first carriage-sliding step before the skin-nicking step and a second carriage-sliding step after the skin-nicking step. The first carriage-sliding step can include distally sliding the nicking-blade carriage 210 including the nicking blade 208 overmolded therein into the nicking position such that the nicking blade 208 extends beyond the distal end of the nose 170 of the RICC insertion device 110. The second carriage-sliding step can include proximally sliding the nicking-blade carriage into the safety position such that the nicking blade 208 is short of the distal end of the nose 170 of the RICC insertion device 110. The skin-nicking step is also performed before the catheter tube-advancing step.

The catheter tube-advancing step can include advancing the catheter tube 116 over the access guidewire 112 and into the blood-vessel lumen. The catheter tube-advancing step can be accomplished, in part, by further splitting the splittable casing 136 away from the longitudinal composite 140 of the RICC assembly 108. The catheter tube 116 can be freed from the longitudinal composite 140 with the splitting of the splittable casing 136, which allows the catheter tube 116 to be advanced over the access guidewire 112 upon removal of any remaining slack of the longitudinal composite 140 in the RICC insertion assembly 102.

The access guidewire-advancing step or the catheter tube-advancing step set forth above can include repeatedly pushing the longitudinal composite 140 into the one-or-more roller wheels 174 disposed in the wheel well 180 of the handle 176 of the RICC insertion device 110 and rolling the longitudinal composite 140 across the one-or-more roller wheels 174 with a single hand to split the splittable casing away from the longitudinal composite 140. The access guidewire-advancing step or the catheter tube-advancing step can additionally or alternatively include pulling the splittable casing 136 of the longitudinal composite 140 out of the split channel 184 formed between the nose 170 of the RICC insertion device 110 and the nose cover 168 thereover to split the splittable casing away from the longitudinal composite 140.

The access guidewire-removing step can include removing the access guidewire 112 leaving the catheter tube 116 in the blood-vessel lumen.

The keeper-removing step can include removing the keeper 114 including the proximal end of the splittable casing 136 attached to the catheter-hub holder 138 from the RICC assembly 108. The keeper-removing step can be accomplished, in part, by removing the catheter hub 118 of the RICC 106 from the catheter-hub holder 138 and splitting any remaining splittable casing 136 of the longitudinal composite 140 away from the catheter tube 116.

The catheter tube-freeing step can include freeing the catheter tube 116 of the RICC 106 from the retaining clamp 188 formed of the nose 170 and the nose cover 168 of the RICC insertion device 110. The catheter tube-freeing step can be accomplished, in part, by rotating the nose cover 168 over the nose 170 to align the longitudinal gap 190 of the nose cover 168 with the through channel 182 in the nose 170 and pulling the catheter tube 116 away from the RICC insertion device 110. The catheter tube-freeing step can be performed with the keeper-removing step leaving only the RICC 106 with the catheter tube 116 disposed in the blood-vessel lumen.

The maneuver guidewire-inserting step can include inserting a maneuver guidewire into the blood-vessel lumen by way of the distal lumen of the RICC 106. The maneuver guidewire of the maneuver guidewire-advancing step can have a length sufficient for advancing the catheter tube 116 of the RICC 106 to a lower ⅓ of the SVC of a heart of the patient. The maneuver guidewire can be part of a guidewire management device configured to maintain sterility of the maneuver guidewire and facilitate the maneuver guidewire-advancing step. Alternatively, the maneuver guidewire can be a stand-alone maneuver guidewire packaged in a sterile barrier (e.g., a bag, a casing, etc.) configured to maintain sterility of the maneuver guidewire. Such a maneuver guidewire can includes a stopping means to stop advancement of the maneuver guidewire during the maneuver guidewire-advancing step, which obviates losing the maneuver guidewire in the patient. The stopping means can be a ball, a slug, or the like coupled to a proximal portion of the maneuver guidewire configured to not pass through, for example, the Luer connector of the extension leg in which at least the proximal portion of the maneuver guidewire is disposed during the maneuver guidewire-advancing step.

The catheter tube-placing step can include advancing the distal portion of the catheter tube 116 farther into the blood-vessel lumen over the maneuver guidewire to the lower ⅓ of the SVC of the heart of the patient.

The maneuver guidewire-withdrawing step can include withdrawing the maneuver guidewire from the blood-vessel lumen of the patient as well from the RICC 106.

### Additional embodiments

FIGS. 29-31, and 32A-32G provide additional embodiments of RICC insertion systems, RICC insertion kits, components thereof, and associated methods, which are not part of the invention, As noted, for convenience, description for particular components of the RICC insertion systems 100 and 300 is set forth herein in sections but such sections are not intended to limit the description for the particular components of the RICC insertion systems 100 and 300. Indeed, description for any component of the RICC insertion system 100 or 300 can crossover from one section into one or more other sections set forth herein in view of the interrelatedness of the components of the RICC insertion systems 100 and 300.

FIGS. 29-31 provide details of a two-stage RICC insertion system 300 including a RICC insertion assembly 302 having an introducer 304 releasably coupled thereto. As shown in FIG. 29, the RICC insertion assembly 302 can include a RICC insertion device 309 having a housing 310 that defines an interior cavity 312 configured to receive one or more of the RICC 106, the access guidewire 112, optionally, together as the RICC assembly 108 but without the keeper 114, as shown, the introducer 304, a needle-retraction assembly 330, a catheter-advancement assembly 340, and a guidewire-advancement assembly 350 therein. As noted, various embodiments of the RICC assemblies 108, 150, 152, 154, and 158, or the like, can be used with the RICC insertion system 300. Similarly, various embodiments of needle, catheter, or guidewire-advancement assemblies as described herein can be used with the RICC insertion system 300.

An outer surface of the housing 310 can define one or more handles configured to facilitate grasping or manipulating the RICC insertion system 300. For example, the RICC insertion device 309 can include a longitudinal handle 315 and a curved portion 317 defined by the housing 310. Advantageously, the interior cavity 312 of the housing 310 can provide a protective environment within which to receive the RICC assembly 108, allowing a user to manipulate the RICC assembly 108 without having to directly contact the RICC assembly 108 or the RICC 106 or access guidewire 112 thereof. In an embodiment, the RICC insertion device 309 can include one or more flexible barriers. One or both of the housing 310 and the one-or-more flexible barriers can co-operate to maintain the RICC assembly 108 within a sterile environment. In an embodiment, the RICC insertion device 309 can include a blood-flash indicator 321 akin to a barrel of a syringe disposed in the housing 310 in fluid communication with a needle lumen of the introducer needle 314 of the introducer 304, as described in more detail herein.

In an embodiment, one or more of the needle-retraction assembly 330, the catheter-advancement assembly 340, or the guidewire-advancement assembly 350 can be disposed within the interior cavity 312 of the housing 310. As shown, the needle-retraction assembly 330 and the catheter-advancement assembly 340 can be disposed within the longitudinal handle 315. The guidewire-advancement assembly 350 can be disposed within the curved portion 317 of the housing 310. However, other configurations of the needle-retraction assembly 330, the catheter-advancement assembly 340, and the guidewire-advancement assembly 350 are also contemplated.

In an embodiment, the needle-retraction assembly 330 can be configured to transition the introducer needle 314 between an extended position and a retracted position, relative to the housing 310. The needle-retraction assembly 330 can include a needle slider 332, extending through a slot disposed in the housing 310 and slidably engaged therewith. The needle slider 332 can be configured to be manipulated by the user to transition the needle-retraction assembly 330 between the extended position and the retracted position.

In an embodiment, the catheter-advancement assembly 340 can be configured to transition the RICC 106 of the RICC assembly 108 between a retracted position and an extended position, relative to the housing 310. The catheter-advancement assembly 340 can include a catheter slider 342, extending through a slot in the housing 310 and slidably engaged therewith. The catheter slider 342 can be configured to be manipulated by the user to transition the catheter-advancement assembly 340 between the extended position and the retracted position. In an embodiment, the catheter-advancement assembly 340 can engage a portion of the catheter hub 118 of the RICC 106. However, it will be appreciated that the catheter-advancement assembly 340 can engage other portions of the RICC 106 without limitation for transition between the retracted position and the extended position.

In an embodiment, the RICC insertion system 300 can further include the guidewire-advancement assembly 350 including a guidewire slider 352. The guidewire-advancement assembly 350 can be configured to transition the access guidewire 112 between an extended position and a retracted position relative to the housing 310. The guidewire slider 352 can extend through the slot in the housing 310 for manipulation by the user to transition the guidewire-advancement assembly 350 between the retracted position and the extended position.

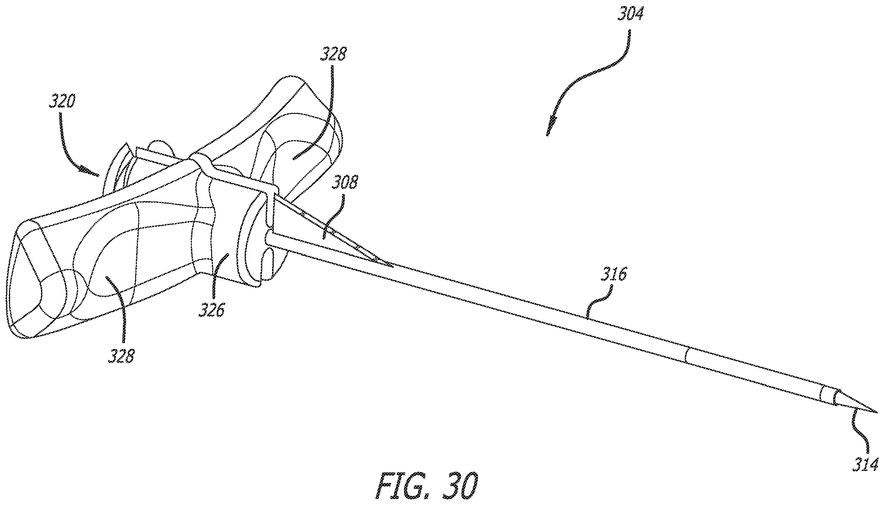

FIG. 30 provides a detailed view of the introducer 304, which can include an introducer needle 314 supported by a needle hub 320 including a needle lumen, as described herein. The introducer 304 can further include a splittable introducer sheath 316 disposed about a needle shaft of the introducer needle 314 and supported at a proximal end by a splittable sheath hub 326, as described herein. The introducer 304 can further include a nicking blade 308 for nicking skin adjacent an access site to widen the access site and allow the RICC 106 to be advanced into a vasculature.

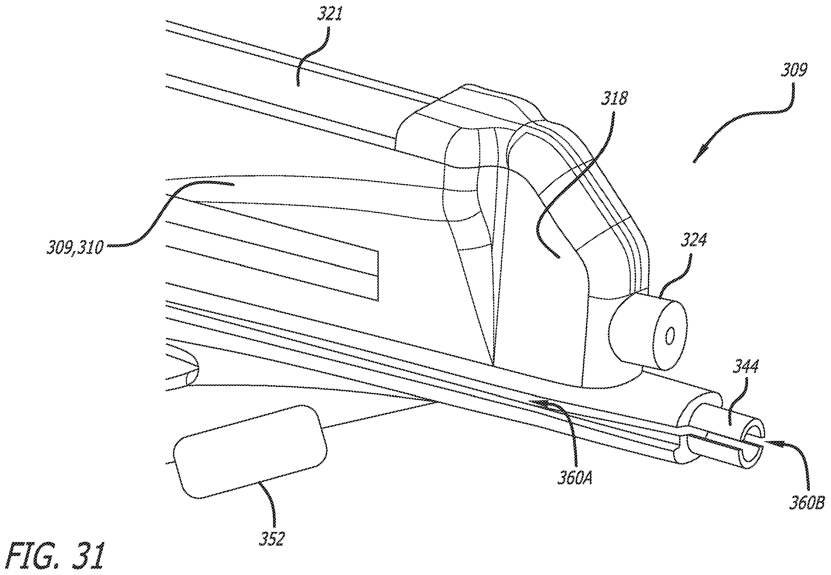

FIG. 31 provides a detailed view of a nose portion 318 of the RICC insertion device 309 including a blood-flash nozzle 324 and a catheter nozzle 344. A proximal end of the introducer 304 can be configured to releasably engage one or both of the blood-flash nozzle 324 and the catheter nozzle 344 in an interference fit, press fit, or snap fit. In an embodiment, one of the needle hub 320 or the sheath hub 326 can be coupled to either the blood-flash nozzle 324 or the catheter nozzle 344, and the needle hub 320 or the sheath hub 326 can provide fluid communication with one or both of the needle lumen and the sheath lumen. For example, when the introducer 304 is coupled with the blood-flash nozzle 324, the needle lumen can be in fluid communication with the blood-flash indicator 321. A fluid flow can be drawn proximally through the needle lumen and into the blood-flash indicator 321 to observe a color and pulsatile flow to confirm correct vascular access. In an embodiment, the blood-flash indicator 321 can include a vacuum disposed therein, or can be configured to create a vacuum therein, to facilitate drawing a proximal fluid flow.

In an embodiment with the introducer 304 coupled with the blood-flash nozzle 324, a distal portion of the needle-retraction assembly 330 can be coupled with the needle hub 320. Actuating the needle-retraction assembly 330 using the needle slider 332 can withdraw the introducer needle 314 into the housing 310 of the RICC insertion device 309. The sheath hub 326 can then be detached from the blood-flash nozzle 324 and coupled with the catheter nozzle 344 to align the lumen of the introducer sheath 316 with the RICC 106, the access guidewire 112, or both the RICC 106 and the access guidewire 112 of the RICC assembly 108. Advantageously, the introducer needle 314 can be withdrawn into the housing 310 after the vasculature has been accessed and be stored in the housing 310, thereby mitigating accidental needle-stick injuries and exposure of body fluids to the user.

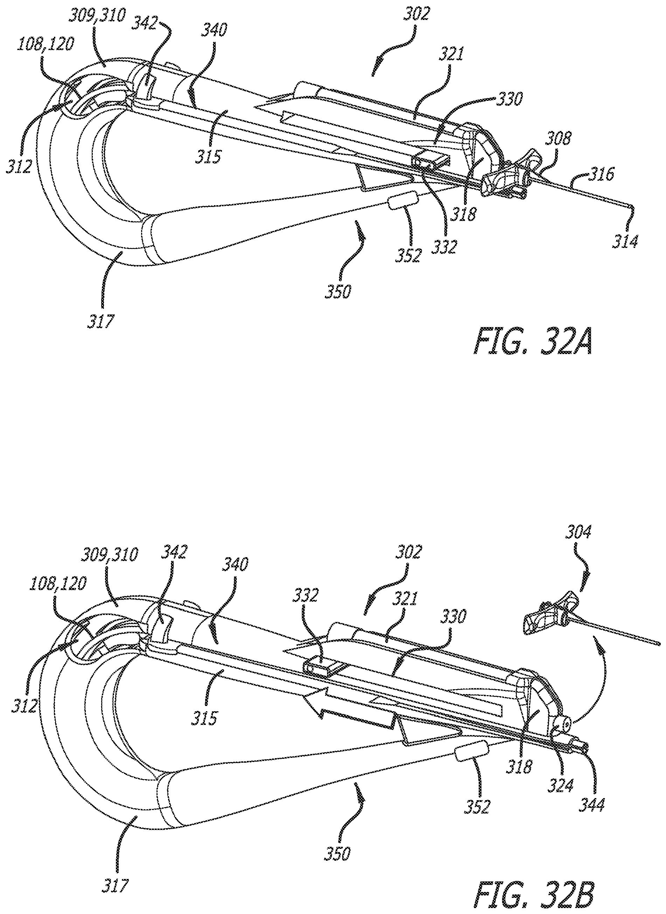

FIGS. 32A-32G provide a different states of the RICC insertion assembly 302 in a method of using the RICC insertion system 300, which is not part of the invention, in accordance with some embodiments.
The RICC insertion system 300 can be provided including the introducer 304 coupled to the blood-flash nozzle 324 and the needle-retraction assembly 330 coupled to the needle hub 320. The needle-retraction assembly 330 can be in the extended position with the introducer needle 314 disposed within the lumen of the introducer sheath 316. A distal needle tip of the introducer needle 314 can be disposed distally of the distal tip of the introducer sheath 316. The RICC insertion assembly 302 can further include the RICC 106 coupled to the catheter-advancement assembly 340, the access guidewire 112 coupled to the guidewire-advancement assembly 350, and the RICC assembly 108 including the RICC 106 and the access guidewire 112 disposed within the housing 310 of the RICC insertion device 309. Both the catheter-advancement assembly 340 and the guidewire-advancement assembly 350 can be in a retracted position with a distal tip of the RICC 106 and a distal tip of the access guidewire 112 disposed within the housing 310.

The user can access a vasculature of the patient by grasping one of the longitudinal handle 315 or the curved portion 317 of the housing 310 to manipulate the RICC insertion assembly 302 and urge the introducer needle 314 distally into the vasculature. As such, the introducer needle 314 and introducer sheath 316 assembly can simultaneously access the vasculature. The blood-flash indicator 321 can draw a fluid flow proximally through the needle lumen and into the blood-flash indicator 321 to confirm correct vascular access.

As shown in FIGS. 32A and 32B, once correct vascular access is confirmed the user can actuate the needle slider 332 to transition the needle-retraction assembly 330 to the retracted position (FIG. 32B) and withdraw the introducer needle 314 from the introducer sheath 316, securing the introducer needle 314 within the housing 310 of the RICC insertion device 309. In an embodiment, the RICC insertion device 309 can then be detached from the introducer 304, or more specifically detached from the sheath hub 326, leaving the introducer sheath 316 in place within the vasculature to maintain patency of the access site. In an embodiment, the user can grasp the sheath hub 326 and advance the introducer sheath 316 further into the access site. The nicking blade 308 disposed proximate the sheath hub 326 can nick the skin adjacent the access site to widen the access site and allow the RICC 106 to be advanced into the vasculature.

Advantageously, detaching the RICC insertion device 309 from the sheath hub 326 allows the user to maintain patency of the access site without having to stabilize the RICC insertion assembly 302 (sans the introducer 104), and any associated components, adjacent the access site. For example, the user can pause the catheter-placement process to prepare subsequent steps, or the like, without having to restart the entire placement process. Further, the blood-flash indicator 321, the introducer needle 314, and the needle-retraction assembly 330 can be off-set from an axis of the catheter-advancement assembly 340, the guidewire-advancement assembly 350, or both, thereby allowing for unobstructed travel of the RICC 106 and the access guidewire 112, as described herein.

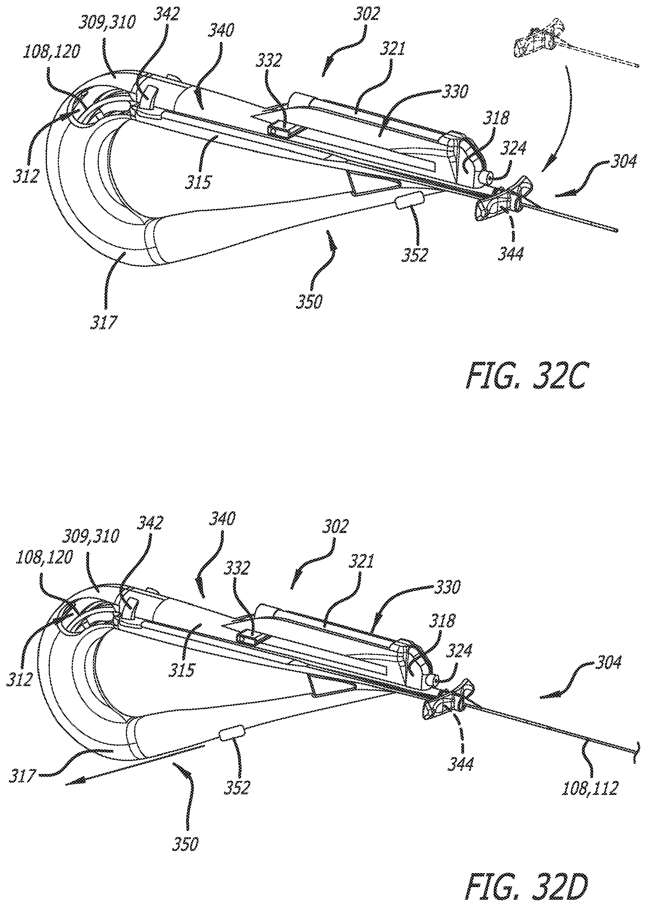

As shown in FIG. 32C, the user can couple the catheter nozzle 344 with the sheath hub 326 to continue the catheter-placement process. Coupling the sheath hub 326 with the catheter nozzle 344 aligns a sheath lumen with the RICC 106, the access guidewire 112, or both. As shown in FIG. 32D, the user can then actuate the guidewire-advancement assembly 350, by manipulating the guidewire slider 352, to transition the access guidewire 112 from the retracted position (FIG. 32C) to the extended position (FIG. 32D). In the extended position, the distal tip of the access guidewire 112 can extend through the primary lumen 124 of the RICC 106, through the catheter nozzle 344 of the housing 310, through the lumen of the introducer sheath 316, and into the vasculature of the patient, until the distal tip of the access guidewire 112 is positioned at a target location.

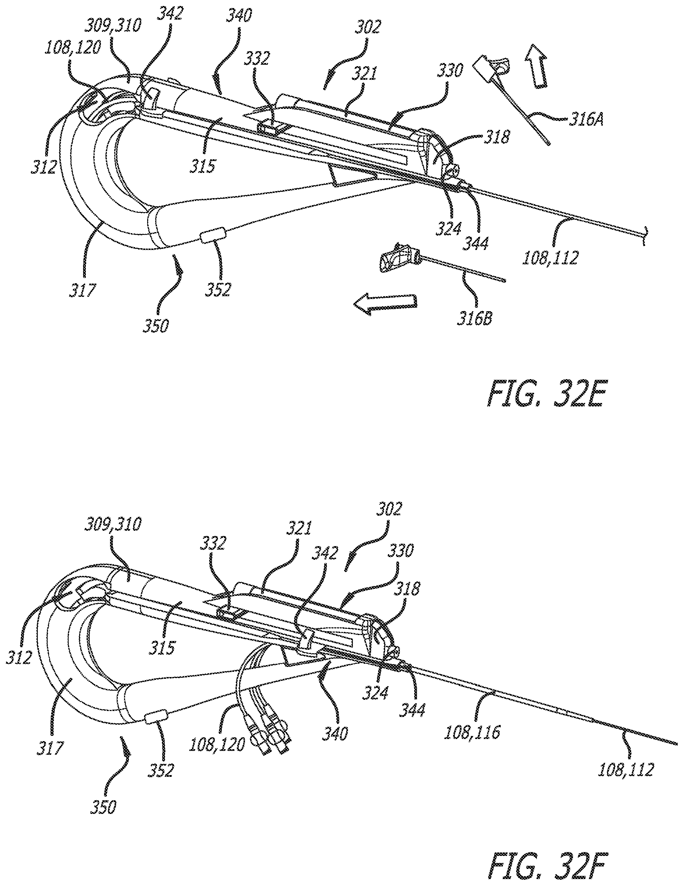

As shown in FIG. 32E, the user can then split the introducer sheath 316 longitudinally into a first portion 316A and a second portion 316B. The user can grasp the wings 328 of the sheath hub 326 and urge them apart laterally to split the sheath hub 326 and the introducer sheath 316. The sheath portions 316A and 316B can then be withdrawn proximally from the access site while the access guidewire 112 maintains patency of the access site. Advantageously, since the inner diameter of the lumen of the introducer sheath 316 is less than an outer diameter of the catheter tube 116 or the RICC 106, the introducer sheath 316 can be split and removed to allow the RICC 106 to be advanced over the access guidewire 112 and into the vasculature.

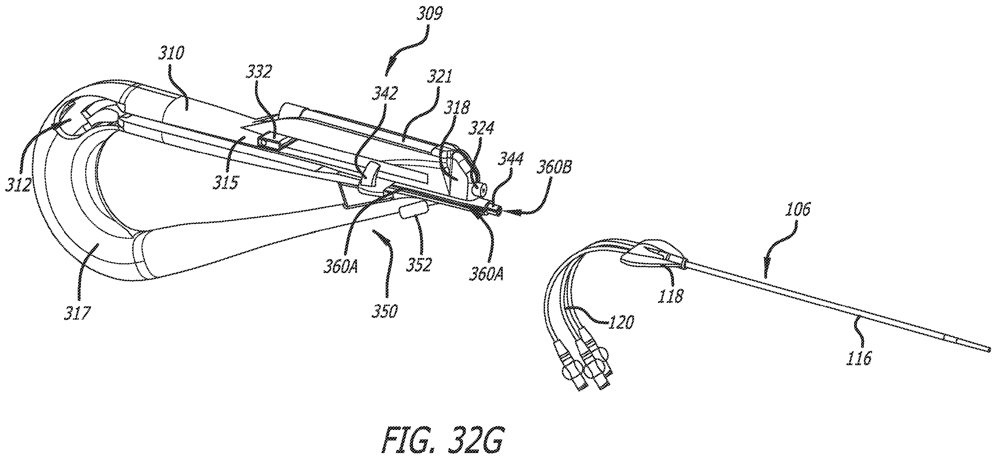

As shown in FIG. 32F, the user can then actuate the catheter slider 342 to transition the catheter-advancement assembly 340 from the retracted position to the extended position. In the extended position, the RICC 106 can be advanced over the access guidewire 112 and into the vasculature. In an embodiment, the housing 310 can include a first lateral slot 360A and a second lateral slot 360B disposed opposite the first lateral slot across a central longitudinal axis of the catheter nozzle 344 (FIG. 31). The first lateral slot 360A and the second lateral slot 360B can extend longitudinally proximally from a distal tip of the catheter nozzle 344 and can communicate with the interior cavity 312 of the housing 310. As such, the first lateral slot 360A and the second lateral slot 360B can allow the catheter hub 118, and the one-or-more extension legs 120 to pass therethrough, allowing the RICC 106 to disengage the housing 310, once placed. As show in FIG. 32G, the guidewire slider 352 can then be retracted to transition the guidewire-advancement assembly 350 and the access guidewire 112 to the retracted position, storing the access guidewire 112 within the housing 310 and mitigating exposure of body fluids to the user.

While some particular embodiments have been disclosed herein, and while the particular embodiments have been disclosed in some detail, it is not the intention for the particular embodiments to limit the scope of the claims provided herein. Additional adaptations or modifications can appear to those of ordinary skill in the art, and, in broader aspects, these adaptations or modifications are encompassed as well. Accordingly, departures may be made from the particular embodiments disclosed herein without departing from the scope of the claims provided herein.

## Claims

1. A rapidly insertable central catheter ("RICC") insertion assembly (102), comprising:
a RICC assembly (108) including:
a RICC (106) including:
a catheter tube (116);
a catheter hub (118) coupled to a proximal portion of the catheter tube (116); and
one or more extension legs (120), each extension leg (120) of the one-or-more extension legs coupled to the catheter hub (118) by a distal portion thereof; **characterised in that**,
it further discloses an access guidewire (112) disposed in a primary lumen (124) of the RICC (106); and
a keeper (114) including a splittable casing (136) over both the catheter tube (116) and a distal portion of the access guidewire (112) extending from a distal end of the RICC (106) forming a longitudinal composite (140) thereof;
a RICC insertion device (110) including:
a frame (172) including:
a longitudinal handle (176) including a wheel well (180);
a nose (170) extending from a distal portion of the handle (176) distal of the wheel well (180), the nose (170) including a through channel (182) configured for advancing the catheter tube (116) of the RICC (106) therethrough; and
a curved cradle (178) extending from a proximal portion of the handle (176);
one or more roller wheels (174) disposed in the wheel well (180); and
a nose cover (168) over the nose (170), the nose cover (168) and nose (170) forming a split channel (184) configured for both splitting and passing the splittable casing (136) therethrough, the RICC insertion device (110) configured for advancing the RICC assembly (108) from an initial position in the frame (172) to a final position in the frame (172) by repeatedly pushing the longitudinal composite (140) into the one-or-more roller wheels (174) and rolling the longitudinal composite (140) across the one-or-more roller wheels (174).

2. The RICC insertion assembly (102) of claim 1, wherein a distal end of the longitudinal composite (140), the splittable casing (136), or the access guidewire (112) is substantially commensurate with a distal end of the frame (172) in the initial position of the RICC assembly (108) in the frame (172), and/or
wherein a slack loop of the longitudinal composite (140) extends away from both the handle (176) and the cradle (178) in the initial position of the RICC assembly (108) in the frame (172).

3. The RICC insertion assembly (102) of either claim 1 or 2, wherein the longitudinal composite (140) is substantially split in the final position of the RICC assembly (108) in the frame (172).

4. The RICC insertion assembly (102) of any claim of claims 1-3, wherein a proximal end of an extension leg (120) or a Luer connector coupled to the extension leg (120) of the one-or-more extension legs of the RICC (106) is substantially commensurate with a proximal end of the cradle (178) in the initial position of the RICC assembly (108) in the frame (172).

5. The RICC insertion assembly (102) of claim 4, wherein a proximal end of the access guidewire (112) is coupled to the proximal end of the cradle (178) in the initial position of the RICC assembly (108) in the frame (172), and/or
wherein the one-or-more extension legs (120) of the RICC (106) lie substantially over the handle (176) in the final position of the RICC assembly (108) in the frame (172).

6. The RICC insertion assembly (102) of any claim of claims 1-5, wherein the catheter hub (118) of the RICC (106) lies over the wheel well (180) in the final position of the RICC assembly (108) in the frame (172).

7. A rapidly insertable central catheter ("RICC") insertion system (100), comprising:
the RICC insertion assembly (102) according to claim 1; and
an introducer (104).

8. The RICC insertion system of claim 7, wherein the keeper (114) further includes a catheter-hub holder (138) to which a proximal end of the splittable casing (136) is attached, the catheter-hub holder (138) configured to hold the catheter hub (118) therein and keep the splittable casing (136) in position over the catheter tube (116) and the access guidewire (112),
preferably wherein the catheter-hub holder (138) includes a perimetrical wall (142) around at least a portion of a perimeter of the catheter-hub holder (138), the perimetrical wall (142) defining a recess into which the catheter hub (118) fits with an engineering fit.

9. The RICC insertion system of claim 7 or 8, wherein the cradle (178) includes a curved extension (198) over a distal portion of the cradle (178), the extension (198) substantially following a same curve as the cradle (178),
preferably wherein the cradle (178) includes an enclosure (192) over the distal portion of the cradle (178) with the extension (198) extending therefrom, the enclosure (192) configured to enclose therein an otherwise exposed proximal portion of the access guidewire (112) in the initial position of the RICC assembly (108) in the frame (172).

10. The RICC insertion system of claim 9, further comprising a retaining clip (202) configured to clip on to a proximal portion of the RICC (106), retain the RICC assembly (108) in the RICC insertion device (110), and keep the catheter tube (116) from prematurely advancing over the access guidewire (112), the retaining clip (202) including a post (206) configured to engage with a slot (200) in the extension (198) that faces an open face of the cradle (178),
preferably wherein the retaining clip (202) is configured to disengage with the slot (200) in the extension (198) when any remaining slack of the longitudinal composite (140) is removed upon advancing the RICC assembly (108) from the initial position to the final position in the frame (172).

11. The RICC insertion system of any claim of claims 7-10, the frame (172) further including a retaining arch (194) over the cradle (178) proximate the proximal portion of the handle (176) from which the cradle (178) extends, the retaining arch (194) configured to retain the RICC assembly (108) in the RICC insertion device (110) and hold the longitudinal composite (140) over the handle (176) as the RICC assembly (108) transitions over the proximal portion of the handle (176) from which the cradle (178) extends upon advancing the RICC assembly (108) from the initial position to the final position in the frame (172), and/or
wherein the nose cover (168) and the nose (170) further form a retaining clamp (188) configured to slidably clamp the longitudinal composite (140) therein, the nose cover (168) rotatable over the nose (170), and the nose cover (168) including a longitudinal gap configured to rotatably align with the through channel (182) in the nose (170) for removal of the longitudinal composite (140), the splittable casing (136), or the catheter tube (116) therefrom.

12. The RICC insertion system of any claim of claims 7-11, further comprising a retractable nicking blade (208), the retractable nicking blade (208) disposed in a nicking-blade carriage (210) slidably integrated into the nose (170), the nicking-blade carriage (210) having a nicking position with the retractable nicking blade (208) extending beyond a distal end of the nose (170) and a safety position with the retractable nicking blade (208) short of the distal end of the nose (170).

13. The RICC insertion system of any claim of claims 7-12, the introducer (104) including:
an introducer needle (214) including:
a needle shaft (218) including a needle tip (222) in a distal portion of the needle shaft (218); and
a needle hub (220) coupled to a proximal portion of the needle shaft (218); and
a splittable introducer sheath (216) configured to accept therein the introducer needle (214), the introducer sheath (216) including:
a splittable sheath body (224); and
a splittable sheath hub (226) coupled to a proximal portion of the sheath body (224), the sheath hub (226) including a pair of outwardly extending wings (328) along a length of the sheath hub (226) having an internal angle of about 90° or less between the wings (328) configured for splitting the sheath hub (226) by pinching the wings (328) together with a single hand,
preferably wherein the sheath hub (226) includes:
a single longitudinal fault along a side of the sheath hub (226) opposite a vertex of the internal angle formed by the wings (328), the sheath hub (226) configured to split along the fault for propagation along a same side of the sheath body (224) when the wings (328) are pinched together; or
a pair of longitudinal faults including a primary fault along a primary side of the sheath hub (226) opposite a vertex of the internal angle formed by the wings (328) and a secondary fault along a secondary side of the sheath hub (226) opposite the primary fault, the sheath hub (226) configured to split along the primary fault for propagation along the primary side of the sheath body (224) when the wings (328) are pinched together, and the sheath hub (226) configured to split along the secondary fault for propagation along the secondary side of the sheath body (224) when the wings (328) are pulled apart.

14. The RICC insertion system of claim 13, the sheath hub (226) further including a valved cap (230) disposed in a proximal portion of the sheath hub (226), the valved cap (230) including a tapered female valved-cap connector (232) and a septum (234) distal of a proximal opening in the female valved-cap connector (232), the female valved-cap connector (232) configured to accept therein a tapered male needle-hub connector extending from a distal portion of the needle hub (220), and the septum (234) configured to accept therethrough the needle shaft (218),
preferably wherein the valved cap (230) is partially or fully split such that the valved cap (230) splits with the sheath hub (226) when the wings (328) are pinched together.

15. The RICC insertion system of claim 13 or 14, the sheath hub (226) further including:
an overmolded nicking blade (236) distally extending from the sheath hub (226); and
a hinged nicking-blade cover (238) having an opened state with the nicking-blade cover (238) opened away from the overmolded nicking blade (236) and a closed state with the nicking-blade cover (238) closed over the nicking blade (236), and/or
the RICC insertion system further comprising:
a syringe (240) including a tapered male syringe tip (242) extending from a distal portion of the syringe (240), the syringe tip (242) configured to insert into a tapered female needle-hub connector in a proximal portion of the needle hub (220).

## Patentansprüche

1. Einführanordnung (102) für einen schnell einführbaren Zentralkatheter ("RICC"), umfassend:
eine RICC-Anordnung (108), einschließend:
einen RICC (106), einschließend:
einen Katheterschlauch (116);
eine Katheternabe (118), die mit einem proximalen Abschnitt des Katheterschlauchs (116) gekoppelt ist; und
einen oder mehrere Verlängerungsschenkel (120), wobei jeder Verlängerungsschenkel (120) von einem oder mehreren Verlängerungsschenkeln mit der Katheternabe (118) durch einen distalen Abschnitt davon gekoppelt ist; **dadurch gekennzeichnet, dass** sie weiter Folgendes offenbart
einen Zugangsführungsdraht (112), der in einem primären Lumen (124) des RICC (106) angeordnet ist; und
eine Fixiervorrichtung (114), die eine teilbare Hülse (136) über sowohl dem Katheterschlauch (116) als auch einem distalen Abschnitt des Zugangsführungsdrahts (112) einschließt, die sich von einem distalen Ende des RICC (106) aus erstreckt und mit diesen einen Längsverbund (140) bildet;
eine RICC-Einführvorrichtung (110), einschließend:
einen Rahmen (172), einschließend:
einen Längsgriff (176), der eine Radmulde (180) einschließt;
eine Nase (170), die sich von einem distalen Abschnitt des Griffs (176) distal zur Radmulde (180) erstreckt, wobei die Nase (170) einen Durchgangskanal (182) einschließt, der zum Vorschieben des Katheterschlauchs (116) des RICC (106) dort hindurch ausgebildet ist; und
eine gekrümmte Aufnahme (178), die sich von einem proximalen Abschnitt des Griffs (176) aus erstreckt;
ein oder mehrere Laufräder (174), die in der Radmulde (180) angeordnet sind; und
eine Nasenabdeckung (168) über der Nase (170), wobei die Nasenabdeckung (168) und die Nase (170) einen geteilten Kanal (184) bilden, der sowohl zum Teilen als auch zum Dorthindurchführen der teilbaren Hülse (136) ausgebildet ist, wobei die RICC-Einführvorrichtung (110) zum Vorschieben der RICC-Anordnung (108) von einer Ausgangsposition im Rahmen (172) in eine Endposition im Rahmen (172) ausgebildet ist, indem der Längsverbund (140) wiederholt in das eine oder die mehreren Laufräder (174) gedrückt wird und der Längsverbund (140) über das eine oder die mehreren Laufräder (174) gerollt wird.

2. RICC-Einführanordnung (102) nach Anspruch 1, wobei ein distales Ende des Längsverbunds (140), der teilbaren Hülse (136) oder des Zugangsführungsdrahts (112) im Wesentlichen einem distalen Ende des Rahmens (172) in der Ausgangsposition der RICC-Anordnung (108) im Rahmen (172) entspricht, und/oder
wobei sich eine lose Schleife des Längsverbunds (140) in der Ausgangsposition der RICC-Anordnung (108) im Rahmen (172) sowohl vom Griff (176) als auch von der Aufnahme (178) weg erstreckt.

3. RICC-Einführanordnung (102) nach Anspruch 1 oder 2, wobei der Längsverbund (140) in der Endposition der RICC-Anordnung (108) im Rahmen (172) im Wesentlichen geteilt ist.

4. RICC-Einführanordnung (102) nach einem der Ansprüche 1-3, wobei ein proximales Ende eines Verlängerungsschenkels (120) oder eines mit dem Verlängerungsschenkel (120) gekoppelten Luer-Verbinders von einem oder mehreren Verlängerungsschenkeln des RICC (106) im Wesentlichen einem proximalen Ende der Aufnahme (178) in der Ausgangsposition der RICC-Anordnung (108) im Rahmen (172) entspricht.

5. RICC-Einführanordnung (102) nach Anspruch 4, wobei ein proximales Ende des Zugangsführungsdrahts (112) in der Ausgangsposition der RICC-Anordnung (108) im Rahmen (172) mit dem proximalen Ende der Aufnahme (178) gekoppelt ist, und/oder
wobei der eine oder die mehreren Verlängerungsschenkel (120) des RICC (106) in der Endposition der RICC-Anordnung (108) im Rahmen (172) im Wesentlichen über dem Griff (176) liegen.

6. RICC-Einführanordnung (102) nach einem der Ansprüche 1-5, wobei die Katheternabe (118) des RICC (106) in der Endposition der RICC-Anordnung (108) im Rahmen (172) über der Radmulde (180) liegt.

7. Einführsystem (100) für einen schnell einführbaren Zentralkatheter ("RICC"), umfassend:
die RICC-Einführanordnung (102) nach Anspruch 1; und
ein Einführinstrument (104).

8. RICC-Einführsystem nach Anspruch 7, wobei die Fixiervorrichtung (114) weiter einen Katheternabenhalter (138) einschließt, an dem ein proximales Ende der teilbaren Hülse (136) angebracht ist, wobei der Katheternabenhalter (138) dazu ausgebildet ist, die Katheternabe (118) darin zu halten und die teilbare Hülse (136) über dem Katheterschlauch (116) und dem Zugangsführungsdraht (112) in Position zu halten,
wobei der Katheternabenhalter (138) vorzugsweise um mindestens einen Abschnitt eines Umfangs des Katheternabenhalters (138) herum eine umlaufende Wand (142) einschließt, wobei die umlaufende Wand (142) eine Aussparung definiert, in die die Katheternabe (118) mit einer technischen Passung passt.

9. RICC-Einführsystem nach Anspruch 7 oder 8, wobei die Aufnahme (178) ein gekrümmtes Verlängerungsstück (198) über einem distalen Abschnitt der Aufnahme (178) einschließt, wobei das Verlängerungsstück (198) im Wesentlichen derselben Krümmung wie die Aufnahme (178) folgt,
wobei die Aufnahme (178) vorzugsweise eine Verkleidung (192) über dem distalen Abschnitt der Aufnahme (178) mit dem sich davon erstreckenden Verlängerungsstück (198) einschließt, wobei die Verkleidung (192) dazu ausgebildet ist, einen ansonsten freiliegenden proximalen Abschnitt des Zugangsführungsdrahts (112) in der Ausgangsposition der RICC-Anordnung (108) im Rahmen (172) zu umschließen.

10. RICC-Einführsystem nach Anspruch 9, weiter umfassend eine Halteklammer (202), die dazu ausgebildet ist, sich an einen proximalen Abschnitt des RICC (106) zu klammern, die RICC-Anordnung (108) in der RICC-Einführvorrichtung (110) zurückzuhalten und den Katheterschlauch (116) daran zu hindern, sich vorzeitig über den Zugangsführungsdraht (112) vorzuschieben, wobei die Halteklammer (202) einen Stift (206) einschließt, der dazu ausgebildet ist, mit einem Schlitz (200) in dem Verlängerungsstück (198) in Eingriff zu kommen, der einer offenen Seite der Aufnahme (178) zugewandt ist,
wobei die Halteklammer (202) vorzugsweise dazu ausgebildet ist, sich aus dem Schlitz (200) in dem Verlängerungsstück (198) zu lösen, wenn beim Vorschieben der RICC-Anordnung (108) von der Ausgangsposition in die Endposition im Rahmen (172) jeglicher verbleibender Spielraum des Längsverbunds (140) beseitigt wird.

11. RICC-Einführsystem nach einem der Ansprüche 7-10, wobei der Rahmen (172) über der Aufnahme (178) in der Nähe des proximalen Abschnitts des Griffs (176), von dem aus sich die Aufnahme (178) erstreckt, weiter einen Haltebogen (194) einschließt, wobei der Haltebogen (194) dazu ausgebildet ist, die RICC-Anordnung (108) in der RICC-Einführvorrichtung (110) zurückzuhalten und den Längsverbund (140) über dem Griff (176) zu halten, während sich die RICC-Anordnung (108) beim Vorschieben der RICC-Anordnung (108) von der Ausgangsposition in die Endposition im Rahmen (172) über den proximalen Abschnitt des Griffs (176), von dem aus sich die Aufnahme (178) erstreckt, hinweg bewegt, und/oder
wobei die Nasenabdeckung (168) und die Nase (170) weiter eine Halteschelle (188) bilden, die dazu ausgebildet ist, den Längsverbund (140) darin verschiebbar festzuklemmen, wobei die Nasenabdeckung (168) über die Nase (170) drehbar ist, und die Nasenabdeckung (168) einen Längsspalt einschließt, der dazu ausgebildet ist, sich zum Entfernen des Längsverbunds (140), der teilbaren Hülse (136) oder des Katheterschlauchs (116) daraus drehbar mit dem Durchgangskanal (182) in der Nase (170) auszurichten.

12. RICC-Einführsystem nach einem der Ansprüche 7-11, weiter umfassend eine einziehbare Einkerbklinge (208), wobei die einziehbare Einkerbklinge (208) in einem Einkerbklingen-Schlitten (210) angeordnet ist, der verschiebbar in die Nase (170) integriert ist, wobei der Einkerbklingen-Schlitten (210) eine Einkerbposition, in der sich die einziehbare Einkerbklinge (208) über ein distales Ende der Nase (170) hinaus erstreckt, und eine Sicherheitsposition, in der die einziehbare Einkerbklinge (208) hinter dem distalen Ende der Nase (170) zurückbleibt, aufweist.

13. RICC-Einführsystem nach einem der Ansprüche 7-12, das Einführinstrument (104), einschließend:
eine Einführinstrumentennadel (214), einschließend:
einen Nadelschaft (218), der in einem distalen Abschnitt des Nadelschafts (218) eine Nadelspitze (222) einschließt; und
eine Nadelnabe (220), die mit einem proximalen Abschnitt des Nadelschafts (218) gekoppelt ist; und
eine teilbare Einführinstrumentenschleuse (216), die dazu ausgebildet ist, die Einführinstrumentennadel (214) darin aufzunehmen, die Einführinstrumentenschleuse (216) einschließend:
einen teilbaren Schleusenkörper (224); und
eine teilbare Schleusennabe (226), die mit einem proximalen Abschnitt des Schleusenkörpers (224) gekoppelt ist, wobei die Schleusennabe (226) ein Paar von sich entlang einer Länge der Schleusennabe (226) nach außen erstreckenden Flügeln (328) einschließt, das einen Innenwinkel von etwa 90° oder weniger zwischen den Flügeln (328) aufweist und dazu ausgebildet ist, dass die Schleusennabe (226) durch Zusammendrücken der Flügel (328) mit nur einer Hand geteilt werden kann,
wobei die Schleusennabe (226) vorzugsweise einschließt:
eine einzige Längsbruchlinie entlang einer Seite der Schleusennabe (226) gegenüber einem Scheitelpunkt des durch die Flügel (328) gebildeten Innenwinkels, wobei die Schleusennabe (226) dazu ausgebildet ist, sich entlang der Bruchlinie zum Ausbreiten entlang derselben Seite des Schleusenkörpers (224) zu teilen, wenn die Flügel (328) zusammengedrückt werden; oder
ein Paar von Längsbruchlinien, die entlang einer primären Seite der Schleusennabe (226) gegenüber einem Scheitelpunkt des durch die Flügel (328) gebildeten Innenwinkels eine primäre Bruchlinie und entlang einer sekundären Seite der Schleusennabe (226) gegenüber der primären Bruchlinie eine sekundäre Bruchlinie einschließen, wobei die Schleusennabe (226) dazu ausgebildet ist, sich entlang der primären Bruchlinie zum Ausbreiten entlang der primären Seite des Schleusenkörpers (224) zu teilen, wenn die Flügel (328) zusammengedrückt werden, und wobei die Schleusennabe (226) dazu ausgebildet ist, sich entlang der sekundären Bruchlinie zu teilen, um sich entlang der sekundären Seite des Schleusenkörpers (224) auszubreiten, wenn die Flügel (328) auseinandergezogen werden.

14. RICC-Einführsystem nach Anspruch 13, wobei die Schleusennabe (226) weiter eine Ventilkappe (230) einschließt, die in einem proximalen Abschnitt der Schleusennabe (226) angeordnet ist, wobei die Ventilkappe (230) einen sich verjüngenden weiblichen Ventilkappenverbinder (232) und ein Septum (234) distal einer proximalen Öffnung im weiblichen Ventilkappenverbinder (232) einschließt, wobei der weibliche Ventilkappenverbinder (232) dazu ausgebildet ist, einen sich verjüngenden männlichen Nadelnabenverbinder darin aufzunehmen, der sich von einem distalen Abschnitt der Nadelnabe (220) erstreckt, und das Septum (234) dazu ausgebildet ist, den Nadelschaft (218) dort hindurch aufzunehmen,
wobei die Ventilkappe (230) vorzugsweise teilweise oder vollständig geteilt ist, sodass sich die Ventilkappe (230) zusammen mit der Schleusennabe (226) teilt, wenn die Flügel (328) zusammengedrückt werden.

15. RICC-Einführsystem nach Anspruch 13 oder 14, wobei die Schleusennabe (226) weiter einschließt:
eine umspritzte Einkerbklinge (236), die sich distal von der Schleusennabe (226) erstreckt; und
eine angelenkte Einkerbklingen-Abdeckung (238), die einen geöffneten Zustand, in dem die Einkerbklingen-Abdeckung (238) von der umspritzten Einkerbklinge (236) weg geöffnet ist, und einen geschlossenen Zustand, in dem die Einkerbklingen-Abdeckung (238) über der Einkerbklinge (236) geschlossen ist, aufweist, und/oder
wobei das RICC-Einführsystem weiter umfasst:
eine Spritze (240), die eine sich verjüngende männliche Spritzenspitze (242) einschließt, die sich von einem distalen Abschnitt der Spritze (240) erstreckt, wobei die Spritzenspitze (242) dazu ausgebildet ist, in einen sich verjüngenden weiblichen Nadelnabenverbinder in einem proximalen Abschnitt der Nadelnabe (220) eingeführt zu werden.

## Revendications

1. Ensemble (102) d'insertion de cathéter central à insertion rapide (« RICC »), comprenant :
un ensemble (108) formant RICC, incluant :
un RICC (106) incluant :
un tube de cathéter (116) ;
une portion cathéter (118) de regroupement, reliée à une portion proximale du tube de cathéter (116) ; et
une ou plusieurs branches d'extension (120), chaque branche d'extension (120) parmi lesdites une ou plusieurs branches d'extension étant reliée, par sa portion distale, à la portion cathéter (118) de regroupement ; **caractérisé en ce que**, il divulgue en outre
un fil-guide d'accès (112), disposé dans une lumière principale (124) du RICC (106) ; et
un élément de maintien (114), incluant une enveloppe (136) apte à être fendue située au-dessus à la fois du tube de cathéter (116) et d'une portion distale du fil-guide d'accès (112) s'étendant depuis une extrémité distale du RICC (106), formant un composite longitudinal (140) de celui-ci ;
un dispositif (110) d'insertion de RICC, incluant :
une structure (172) incluant :
une poignée longitudinale (176), incluant un logement (180) de galets ;
un nez (170), s'étendant à partir d'une portion distale de la poignée (176), de manière distale par rapport au logement (180) de galets, le nez (170) incluant un canal traversant (182) configuré de manière à faire avancer le tube de cathéter (116) du RICC (106) à travers lui ; et
un berceau incurvé (178), s'étendant à partir d'une portion proximale de la poignée (176) ;
un ou plusieurs galets (174), disposés dans le logement (180) de galets ; et
un couvercle (168) de nez, placé sur le nez (170), le couvercle (168) de nez et le nez (170) formant un canal fendu (184) configuré à la fois pour le fendage de l'enveloppe (136) apte à être fendue et pour le passage de cette dernière à travers lui, le dispositif (110) d'insertion de RICC étant configuré de manière à faire avancer l'ensemble (108) formant RICC d'une position initiale dans la structure (172) jusqu'à une position finale dans la structure (172), par le fait de pousser de façon répétée le composite longitudinal (140) jusque sur lesdits un ou plusieurs galets (174) et par le fait de faire rouler le composite longitudinal (140) sur lesdits un ou plusieurs galets (174).

2. Ensemble (102) d'insertion de RICC selon la revendication 1, dans lequel une extrémité distale du composite longitudinal (140), de l'enveloppe (136) apte à être fendue, ou du fil-guide d'accès (112) est sensiblement au même niveau qu'une extrémité distale de la structure (172) dans la position initiale de l'ensemble (108) formant RICC dans la structure (172), et/ou
dans lequel une boucle de mou du composite longitudinal (140) s'étend depuis à la fois de la poignée (176) et le berceau (178), en s'éloignant de ceux-ci, dans la position initiale de l'ensemble (108) formant RICC dans la structure (172).

3. Ensemble (102) d'insertion de RICC selon l'une ou l'autre des revendications 1 ou 2, dans lequel le composite longitudinal (140) est sensiblement fendu dans la position finale de l'ensemble (108) formant RICC dans la structure (172).

4. Ensemble (102) d'insertion de RICC selon une revendication quelconque parmi les revendications 1 à 3, dans lequel une extrémité proximale d'une branche d'extension (120) ou d'un connecteur Luer relié à la branche d'extension (120) desdites une ou plusieurs branches d'extension du RICC (106) est sensiblement au même niveau qu'une extrémité proximale du berceau (178) dans la position initiale de l'ensemble (108) formant RICC dans la structure (172).

5. Ensemble (102) d'insertion de RICC selon la revendication 4, dans lequel une extrémité proximale du fil-guide d'accès (112) est reliée à l'extrémité proximale du berceau (178) dans la position initiale de l'ensemble (108) formant RICC dans la structure (172), et/ou
dans lequel lesdites une ou plusieurs branches d'extension (120) du RICC (106) sont situés sensiblement au-dessus de la poignée (176) dans la position finale de l'ensemble (108) formant RICC dans la structure (172).

6. Ensemble (102) d'insertion de RICC selon une revendication quelconque parmi les revendications 1 à 5, dans lequel la portion cathéter (118) de regroupement du RICC (106) est située au-dessus du logement (180) de galets dans la position finale de l'ensemble (108) formant RICC dans la structure (172).

7. Système (100) d'insertion de cathéter central à insertion rapide (« RICC »), comprenant :
l'ensemble (102) d'insertion de RICC selon la revendication 1 ; et
un introducteur (104).

8. Système d'insertion de RICC selon la revendication 7, dans lequel l'élément de maintien (114) inclut en outre un support (138) de portion cathéter de regroupement, auquel une extrémité proximale de l'enveloppe (136) apte à être fendue est fixée, le support (138) de portion cathéter de regroupement étant configuré de manière à maintenir en lui la portion cathéter (118) de regroupement et à maintenir l'enveloppe (136) apte à être fendue en position sur le tube de cathéter (116) et le fil-guide d'accès (112),
dans lequel, de préférence, le support (138) de portion cathéter de regroupement inclut une paroi périphérique (142) autour d'au moins une partie d'un périmètre du support (138) de portion cathéter de regroupement, la paroi périphérique (142) définissant un évidement dans lequel la portion cathéter (118) de regroupement est insérée avec engagement ajusté.

9. Système d'insertion de RICC selon la revendication 7 ou la revendication 8, dans lequel le berceau (178) inclut une extension incurvée (198) sur une portion distale du berceau (178), l'extension (198) suivant sensiblement une même courbe que le berceau (178),
dans lequel, de préférence, le berceau (178) inclut un boîtier (192) sur la portion distale du berceau (178), l'extension (198) s'étendant à partir de celui-ci, le boîtier (192) étant configuré de manière à enfermer en lui une portion proximale autrement exposée du fil-guide d'accès (112) dans la position initiale de l'ensemble (108) formant RICC dans la structure (172).

10. Système d'insertion de RICC selon la revendication 9, comprenant en outre un clip de retenue (202), configuré de manière à se clipser sur une portion proximale du RICC (106), à retenir l'ensemble (108) formant RICC dans le dispositif (110) d'insertion de RICC, et à empêcher le tube de cathéter (116) d'avancer prématurément sur le fil-guide d'accès (112), le clip de retenue (202) incluant une saillie (206) configurée de manière à venir en engagement avec une fente (200) présente dans l'extension (198) qui est en face d'une face ouverte du berceau (178),
dans lequel, de préférence, le clip de retenue (202) est configuré de manière à venir en désengagement d'avec la fente (200) dans l'extension (198) lorsque tout mou restant du composite longitudinal (140) est supprimé lors de l'avancement de l'ensemble (108) formant RICC, de la position initiale à la position finale, dans la structure (172).

11. Système d'insertion de RICC selon une revendication quelconque parmi les revendications 7 à 10, la structure (172) incluant en outre un arceau de retenue (194) au-dessus du berceau (178) à proximité de la portion proximale de la poignée (176) à partir de laquelle le berceau (178) s'étend, l'arceau de retenue (194) étant configuré de manière à retenir l'ensemble (108) formant RICC dans le dispositif (110) d'insertion de RICC et à maintenir le composite longitudinal (140) au-dessus de la poignée (176) lorsque l'ensemble (108) formant RICC passe au-dessus de la portion proximale de la poignée (176) à partir de laquelle le berceau (178) s'étend lors de l'avancement de l'ensemble (108) formant RICC, de la position initiale à la position finale, dans la structure (172), et/ou
dans lequel le couvercle (168) de nez et le nez (170) forment en outre une pince de retenue (188) configurée pour pincer en elle, de manière coulissante, le composite longitudinal (140), le couvercle (168) de nez étant apte à pivoter sur le nez (170), et le couvercle (168) de nez incluant un espace longitudinal configuré de manière à, par rotation, venir en alignement avec le canal traversant (182) présent dans le nez (170) pour le retrait, par rapport à lui-même, du composite longitudinal (140), de l'enveloppe (136) apte à être fendue, ou du tube de cathéter (116).

12. Système d'insertion de RICC selon une revendication quelconque parmi les revendications 7 à 11, comprenant en outre une lame d'incision rétractable (208), la lame d'incision rétractable (208) étant disposée dans un coulisseau (210) porte-lame d'incision intégré de manière coulissante dans le nez (170), le coulisseau (210) porte-lame d'incision ayant une position d'incision, avec la lame d'incision rétractable (208) s'étendant au-delà d'une extrémité distale du nez (170), et une position de sécurité, avec la lame d'incision rétractable (208) située en deçà de l'extrémité distale du nez (170).

13. Système d'insertion de RICC selon une revendication quelconque parmi les revendications 7 à 12, l'introducteur (104) incluant :
une aiguille (214) d'introducteur, incluant :
une tige (218) d'aiguille, incluant une pointe (222) d'aiguille dans une portion distale de la tige (218) d'aiguille ; et
une portion (220) de montage d'aiguille, reliée à une portion proximale de la tige (218) d'aiguille ; et
une gaine d'introducteur (216) apte à être fendue, configurée de manière à accepter en elle l'aiguille (214) d'introducteur, la gaine d'introducteur (216) incluant :
un corps de gaine (224) apte à être fendue ; et
une portion (226) de montage de gaine apte à être fendue, relié à une portion proximale du corps de gaine (224), la portion (226) de montage de gaine incluant une paire d'ailettes (328) s'étendant vers l'extérieur le long d'une longueur de la portion (226) de montage de gaine et ayant un angle intérieur d'environ 90° ou moins entre les ailettes (328), configurées pour le fendage de la portion (226) de montage de gaine, par pincement des ailettes (328) ensemble avec une seule main,
dans lequel, de préférence, la portion (226) de montage de gaine inclut :
une seule ligne de faiblesse longitudinale le long d'un côté de la portion (226) de montage de gaine opposé à un sommet de l'angle intérieur formé par les ailettes (328), la portion (226) de montage de gaine étant configuré pour fendre le long de la ligne de faiblesse avec propagation le long d'un même côté du corps de gaine (224) lorsque les ailettes (328) sont pincées ensemble ; ou
une paire de lignes de faiblesse longitudinales, incluant une ligne de faiblesse primaire le long d'un côté primaire de la portion (226) de montage de gaine opposé à un sommet de l'angle intérieur formé par les ailettes (328) et une ligne de faiblesse secondaire le long d'un côté secondaire de la portion (226) de montage de gaine opposé à la ligne de faiblesse primaire, la portion (226) de montage de gaine étant configurée de manière à fendre le long de la ligne de faiblesse primaire pour une propagation le long du côté primaire du corps de gaine (224) lorsque les ailettes (328) sont pincées ensemble, et la portion (226) de montage de gaine étant configurée de manière à fendre le long de la ligne de faiblesse secondaire pour une propagation le long du côté secondaire du corps de gaine (224) lorsque les ailettes (328) sont écartées.

14. Système d'insertion de RICC selon la revendication 13, la portion (226) de montage de gaine incluant en outre un capuchon valvé (230), disposé dans une portion proximale de la portion (226) de montage de gaine, le capuchon valvé (230) incluant un connecteur femelle conique (232) de capuchon valvé et un septum (234), situé distalement par rapport à une ouverture proximale dans le connecteur femelle (232) de capuchon valvé, le connecteur femelle (232) de capuchon valvé étant configuré de manière à accepter en lui un connecteur mâle conique de montage d'aiguille s'étendant à partir d'une portion distale de la portion (220) de montage d'aiguille, et le septum (234) étant configuré de manière à accepter la tige d'aiguille (218) à travers lui,
dans lequel, de préférence, le capuchon valvé (230) est partiellement ou entièrement fendu de telle sorte que le capuchon valvé (230) se fend avec la portion (226) de montage de gaine lorsque les ailettes (328) sont pincées ensemble.

15. Système d'insertion de RICC selon la revendication 13 ou la revendication 14, la portion (226) de montage de gaine incluant en outre :
une lame d'incision surmoulée (236), s'étendant en position distale à partir de la portion (226) de montage de gaine ; et
un couvercle articulé (238) de lame d'incision, ayant un état ouvert, avec le couvercle (238) de lame d'incision ouvert situé à distance de la lame d'incision surmoulée (236), et un état fermé, avec le couvercle (238) de lame d'incision fermé sur la lame d'incision (236), et/ou
le système d'insertion de RICC comprenant en outre :
une seringue (240), incluant un embout mâle conique (242) de seringue, s'étendant à partir d'une portion distale de la seringue (240), l'embout de seringue (242) étant configuré de manière à s'insérer dans un connecteur femelle conique de montage d'aiguille dans une partie proximale de la portion (220) de montage d'aiguille.
